# EUROPEAN PATENT APPLICATION

(11) **EP 1 782 686 A1**
(43) Date of publication of application: **09.05.2007**
(21) Application number: 06021089.5
(22) Date of filing: 06.10.2006
(51) Int. Cl.: A01H 5/00

(54) **Use of willow for phytoremediation of arsenic contaminated substrate**

(30) Priority: 06.10.2005 US 244988; 06.10.2005 US 244986; 06.10.2005 US 244636; 06.10.2005 US 244975; 06.10.2005 US 244987; 06.10.2005 US 244842; 06.10.2005 US 244635; 06.10.2005 US 800907 P
(71) Applicant: The Research Foundation of the State University of New York, Albany, NY 12207 (US)
(72) Inventor: Abrahamson, Lawrence P., Marcellus NY 13108 (US); Kopp, Richard F., Marietta NY 13110 (US); Smart, Lawrence B., Geneva NY14456 (US); Volk, Timothy A., Syracuse NY 13224 (US); Purdy, Jason J., Syracuse NY 13208 (US)
(74) Representative: Hauck Patent- und Rechtsanwälte

(57) **Abstract**

Use of shrub willow for taking up and accumulating arsenic from contaminated growth medium.

## Description

### FIELD OF THE INVENTION

The invention relates to systems and methods of removing contaminants from soil and water in general and particularly to a phytoremediation system and method that employs shrub willows to achieve contaminant removal while providing biomass.

### DESCRIPTION OF THE INVENTION

An inventive method is set forth in claim 1. Further advantages and aspects thereof are subject of the subclaims.

According to the invention, shrub willow is used for taking up and accumulating arsenic from contaminated growth medium. Surprisingly, shrub willow has the ability to take and accumulate arsenic from contaminated growth medium, especially contaminated soil or water. This can be used to remove contaminants from soil, water or other growth medium. The efficiency of shrub willow is more less pronounced, depending from the shrub willow variety.

Within the present invention the inventors have identified shrub willow varieties produced through controlled breeding at SUNY-ESF that have the ability to take up and accumulate arsenic from contaminated soil or water in their stems and leaves. The arsenic accumulated in the stems and leaves could then be harvested and removed from the contaminated site. In one embodiment, the contaminated shrub willow can be disposed of in a secure landfill. In another embodiment, the contaminated shrub willow can be burned as biomass, and the contaminant (for example arsenic) can then be collected in the ash remaining after incineration of the plant tissues in a properly equipped facility, and/or can be scrubbed from the effluent gases generated during the burning of the biomass. The inventors have demonstrated that one of these varieties, 'Tully Champion' (ESF clone ID# 99202-011), has the ability to accumulate as much as 329 µg As per gram in leaf tissue and 200 µg As per gram in stem tissue with the addition of phosphorus. A second variety, 'Canastota' (clone I.D. 9970-036) also demonstrated the capacity to accumulate high amounts of arsenic, with leaf and stem concentrations as high as 109 and 94 µg As per gram, respectively, in the presence of phosphorus. The addition of phosphorus greatly enhanced arsenic uptake. Both of these varieties are capable of producing upwards of 6 oven-dried tons of biomass per acre per year on uncontaminated land. With the ability to tolerate high levels of arsenic, these varieties offer the possibility of growing biomass for energy production on arsenic contaminated land as well. The identification of shrub willow varieties with the ability to accumulate high levels of arsenic in harvestable tissues will provide a new and more cost-effective alternative for the remediation of arsenic-contaminated land and water.

The following disclosure concern the above and further aspects, details and examples of the prior art and the systems and methods according to the invention:
The clone identification of preferred shrub willow varieties is as follows:
   A = ESF clone ID# 99113-012 (*Salix purpurea*)
   B = 'Tully Champion' (ESF clone ID# 99202-011, *Salix viminalis x S. miyabeana*)
   C = 'Canastota' (ESF clone ID# 9970-036 *Salix sachalinensis x S. miyabeana)*
   D = ESF clone ID# 00x-026-082 (*Salix eriocephala*)
**Hydroponic screening of willow varieties for arsenic uptake. J. Purdy and L.B. Smart. Environmental and Forest Biology. SUNY College of Environmental Science and Forestry. Syracuse, NY 13210. U.S.A.**
Pollution of the environment has increased dramatically over the last century.
Past activities, such as industrial chemical processing, energy and fuel production, mining and smelting processes and pesticide application have scattered more than 450,000 brownfields (G.A.O., 1987) and 3,000 heavily-contaminated sites across the U.S. (E.P.A., 2003). Despite efforts to reduce further pollution of our land and water, the U.S. continues to produce 40 million tons of hazardous waste annually (E.P.A., 2003). Remediation of contaminated sites using conventional methods is projected to cost $400 billion over the coming years in the U.S. alone (Salt *et al.,* 1995).
Arsenic (As) contamination has become a major concern in many parts of the world due to its pervasiveness and persistence in the environment. Arsenic is a highly toxic trace metalloid that is found in virtually all environmental media. Exposure to elevated levels of arsenic can cause skin lesions, lung, kidney, and liver cancers, and damage to the nervous system. Forty-one percent of the U.S. Superfund sites are contaminated with arsenic, which is classified as a group A human carcinogen and listed among the highest priority contaminants on the ATSDR/EPA priority list of hazardous substances (ATSDR, 2005). More than 10,000 As-contaminated sites have been reported in Australia along with thousands of sites across Europe (Fitz and Wenzel, 2002).
High concentrations of arsenic are naturally present in some soils as a result of alluvial deposition and weathering of arsenic-rich parent materials. Arsenic is generally found at higher concentrations in sedimentary rocks and is commonly associated with sulfide deposits. Mean sediment arsenic concentrations range from 5 to 3000 mg kg⁻¹, with the higher levels occurring in areas of contamination. Background concentrations in soil range from 1 to 40 mg kg⁻¹, with mean values around 5 mg kg⁻¹ (Matschullat, 2000). Perhaps the best known case of natural arsenic contamination is in Bangladesh where more than 80 million people suffer from arsenic poisoning due to drinking contaminated water.
Major anthropogenic sources of arsenic include mining and smelting processes and the use of chromated copper arsenate (CCA) as a wood preservative and pesticide for pressure-treated lumber. When used as a preservative, CCA easily leaches into surrounding soils and is directly responsible for unusually high levels of arsenic in many backyards and playgrounds. Organic and inorganic arsenical pesticides also represent a significant source of arsenic pollution. In the past, as much as 20,000 tons of arsenical pesticides were sprayed on crops in the U.S. each year (Fitz and Wenzel, 2002). While inorganic arsenicals such as lead arsenate are, for the most part, no longer used, they do persist in fields and orchards where they were applied years before.
**Phytoremediation**
Phytoremediation, the use of plants to extract, degrade, or immobilize environmental contaminants, has been gaining considerable attention over recent years as a cost-effective alternative to traditional remedial strategies. Currently available technologies such as excavation, burial, acid washing, and incineration are not only costly and time-consuming, but they often destroy the biological component of soil and drastically alter its chemical and physical properties creating a nonviable waste. In contrast, plant-based technologies entail minimal environmental disturbance, require little maintenance or energy input, produce little or no byproduct, and leave the soil intact for future use. Moreover, plants are often capable of remediating contaminated sites at only 10 to 50% of the cost of mechanical, thermal, or chemical treatments (Flathman and Lanza, 1998).
While many plants have been screened for their ability to remove or contain contaminants, the majority of plants used in these studies were not specifically bred for this purpose. Shrub willow *(Salix* spp.) are particularly well-suited for phytoremediation applications due to their substantial biomass production, ease of propagation, high transpiration rates, wide genetic and habitat diversity, and ability to colonize harsh or disturbed sites. In addition, shrub willow can be grown in short rotation coppice culture, in which aboveground biomass is harvested at regular intervals and used as a source of renewable energy (White *et al.,* 1999).
The research performed thus far on the use of willow for phytoremediation suggests that there is great potential for *Salix* spp in remediation systems. Studies have demonstrated that willow is not only capable of tolerating a number of pollutants, but can also accumulate or degrade several contaminants including cadmium, zinc, copper, and ferrocyanide (Landberg and Greger, 1996; Pulford *et al.,* 2002; Ebbs *et al.,* 2003;
Kuzovkina *et al.,* 2004). This project takes advantage of the extensive *Salix* genetic resources accumulated by researchers at SUNY-ESF over the last 15 years (Kopp *et al.,* 2001; Smart *et al.,* 2005) to identify shrub willow varieties that are well-suited for arsenic remediation.
The objectives of this study were to screen a number of shrub willow clones for differences in arsenic tolerance and accumulation quickly using hydroponic culture, determine in what tissues arsenic accumulates, and assess the influence of phosphorus on arsenic uptake.
**Screening shrub willow for As uptake**
**Methods**
Dormant stem cuttings of four shrub willow clones were rooted and grown for four weeks in 0.25x modified Hoagland's solution. After four weeks, the Hoagland's solution was replaced with one of four solutions: 0.25x Hoagland's minus P (-P), 0.25x Hoagland's minus P plus 100 µM sodium arsenate (As100 -P), 0.25x Hoagland's minus P plus 250 µM sodium arsenate (As250 -P), and 0.25x Hoagland's plus 250 µM sodium arsenate (As250+P). With the exception of treatment As250+P, phosphate was excluded from nutrient solutions due to the tendency of this compound to interfere with arsenic uptake. Treatment solutions were replaced weekly over a three-week period. After three weeks, plants were harvested and separated into roots, stems, leaves, and original cuttings, and then dried and weighed. Plant tissues were microwave digested and analyzed using ICP-OES to quantify As and P uptake. Biomass production and transpiration rates were used as measures of tolerance.
**Results**
The effects of arsenic on plant growth differed significantly among the clones used in this study (Table 1). Clones B and C were particularly tolerant of arsenic, demonstrated by the ability of each to maintain biomass production and transpiration rates equal to that of the control in the presence of arsenic. In addition to being more tolerant, these clones overall had greater biomass production and transpiration rates than clones A and D across all treatments. Overall, clone A performed relatively poorly even in the absence of arsenic, which may suggest that this particular variety had difficulty tolerating the saturated conditions of this hydroponic system. In the absence of

**Table 1. Differences in arsenic tolerance among clones**

| **clone** | **leaf biomass (g)** | | | | **stem biomass (g)** | | | | **transpiration (L)** | | | |
|---|---|---|---|---|---|---|---|---|---|---|---|---|
| | **control (-P)** | **As100 (-P)** | **As250 (-P)** | **As250 +P** | **control (-P)** | **As100 (-P)** | **As250 (-P)** | **As250 +P** | **control (-P)** | **As100 (-P)** | **As250 (-P)** | **As250 +P** |
| **A** | 8.88 | 5.28 | 3.22 | 7.55 | 7.67 | 5.73 | 3.75 | 7.19 | 3.75 | 1.93 | 1.27 | 3.09 |
| | A c | AB b | B c | AB b | A b | A b | A b | A c | A b | AB b | B b | A b |
| **B** | 16.42 | 14.51 | 15.75 | 15.26 | 15.60 | 10.54 | 13.89 | 16.36 | 7.15 | 5.91 | 6.92 | 8.32 |
| | A b | A a | A a | A a | A a | B a | AB a | A a | A a | A a | A a | A a |
| **C** | 16.56 | 13.66 | 11.31 | 19.53 | 10.48 | 9.93 | 8.06 | 13.61 | 5.80 | 5.61 | 4.06 | 7.95 |
| | AB b | B a | B ab | A a | AB b | AB a | B b | A ab | AB ab | AB a | B ab | A a |
| **D** | 22.78 | 7.61 | 8.29 | 17.19 | 16.23 | 4.23 | 5.20 | 11.05 | 8.80 | 1.71 | 1.88 | 5.67 |
| | A a | C b | C bc | B a | A a | C b | C b | B bc | A a | C b | C b | B ab |

| | | | | | | | | | | | | |
|---|---|---|---|---|---|---|---|---|---|---|---|---|
| * For each parameter, values with the same capital letter within the same row are not significantly different. Values with the same lowercase letter within the same column are not significantly different (α = .05, n = 4) Clone designations: A = *Salix purpurea,* B = *S. viminalis x S. miyabeana,* C = *S. sachalinensis x S. miyabeana.* D = *S. eriocephala* | | | | | | | | | | | | |

**Table 2. Arsenic concentrations in leaves and stems**

| **clone** | **leaves** | | | | **stems** | | | |
|---|---|---|---|---|---|---|---|---|
| | **control (-P)** | **As100 (-P)** | **As250 (-P)** | **As250 +P** | **control (-P)** | **As100 (-P)** | **As250 (-P)** | **As250 +P** |
| **A** | * | 8.13 | 11.96 | 26.75 | * | 3.55 | 5.88 | 8.55 |
| | | A a | A a | A b | | A a | A a | A c |
| **B** | * | 24.43 | 45.33 | 150.85 | * | 13.60 | 23.75 | 82.03 |
| | | B a | B a | A a | | B a | B a | A a |
| **C** | * | 10.30 | 16.93 | 62.98 | * | 12.23 | 20.60 | 49.88 |
| | | B a | AB a | A b | | B a | AB a | A ab |
| **D** | * | 30.15 | 47.58 | 61.89 | * | 21.63 | 37.15 | 25.68 |
| | | A a | A a | A b | | A a | A a | A bc |

| | | | | | | | | |
|---|---|---|---|---|---|---|---|---|
| * For each tissue, values with the same capital letter within the same row are not significantly different. Values with the same lowercase letter within the same column are not significantly different (α = .05, n = 4) | | | | | | | | |

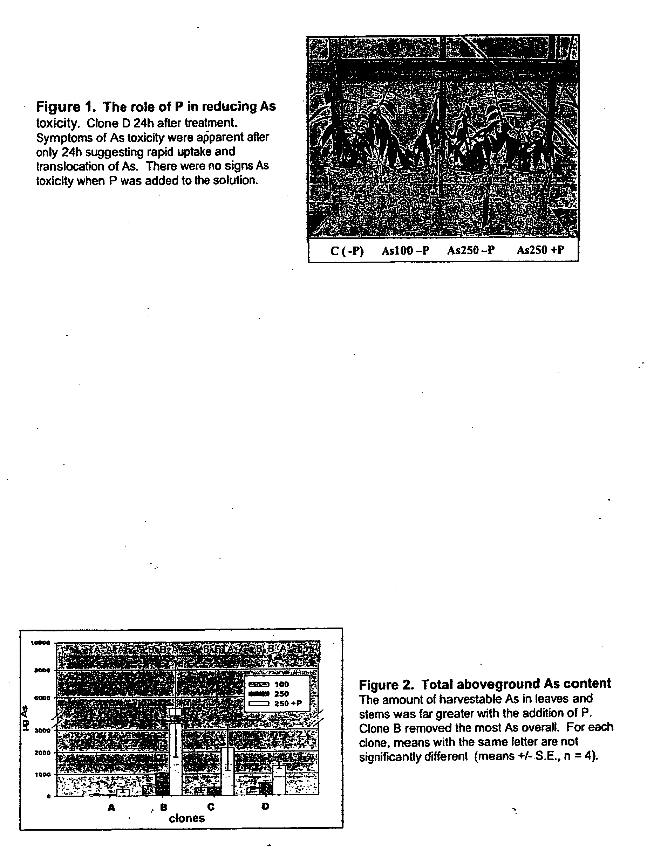

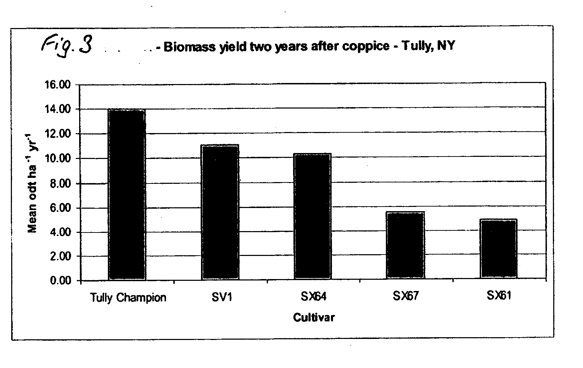

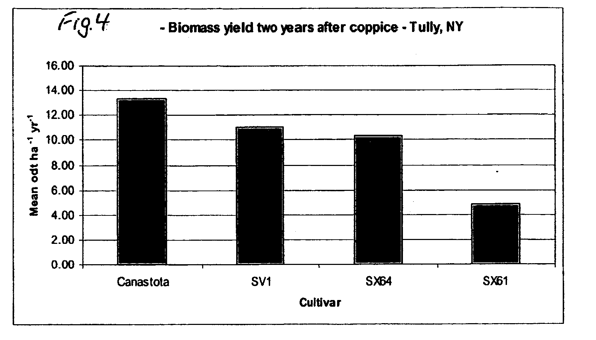

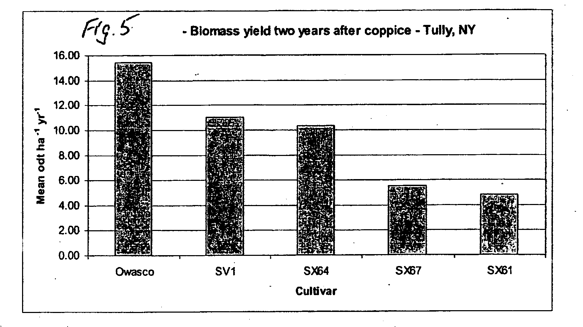

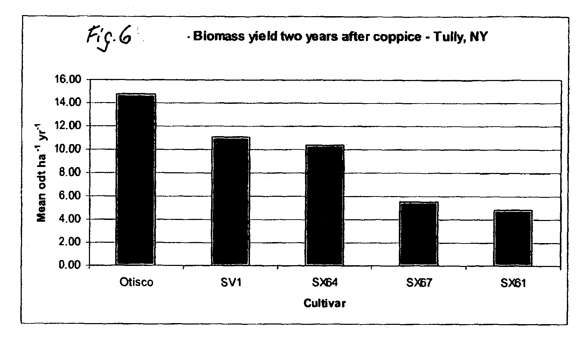

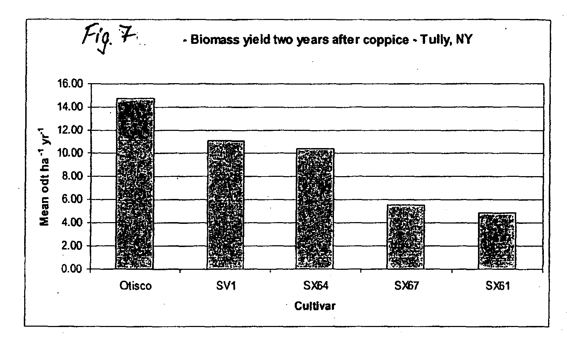

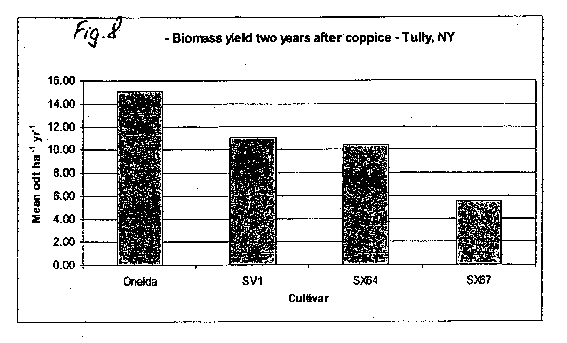

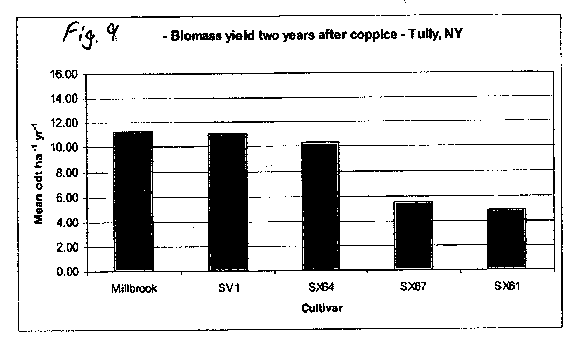

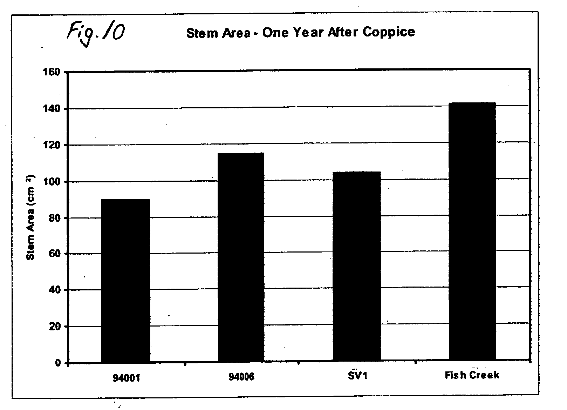

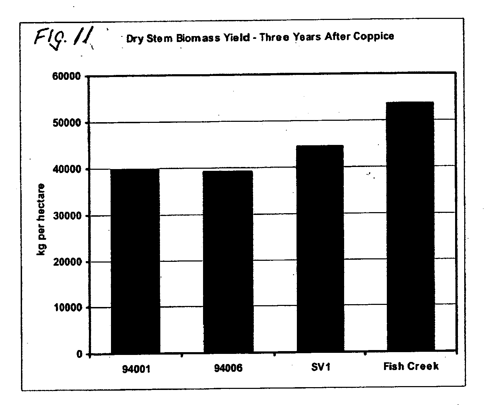

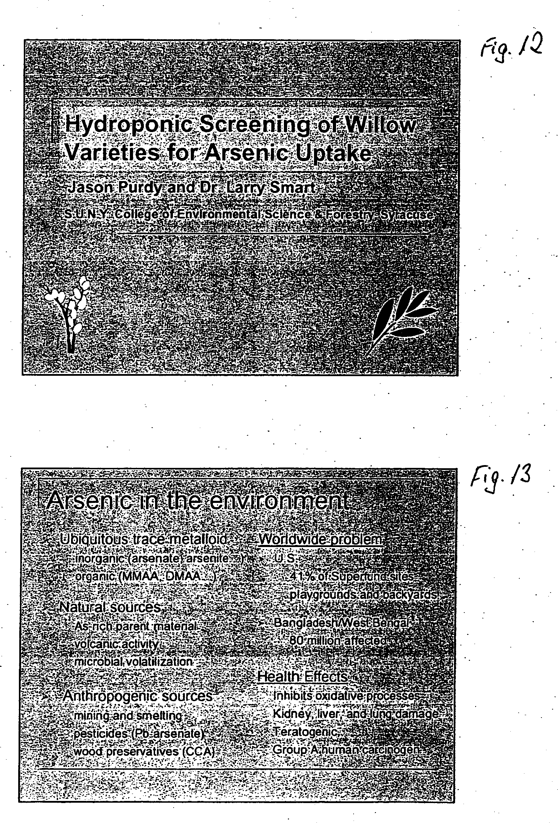

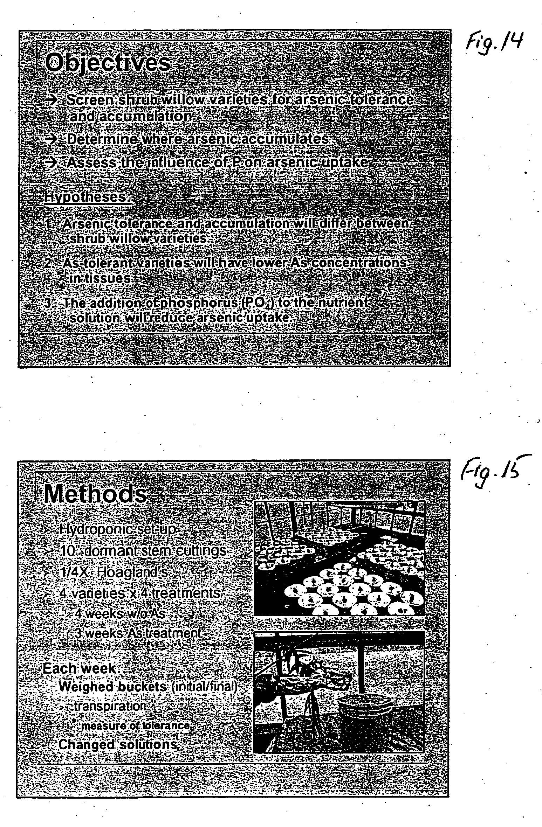

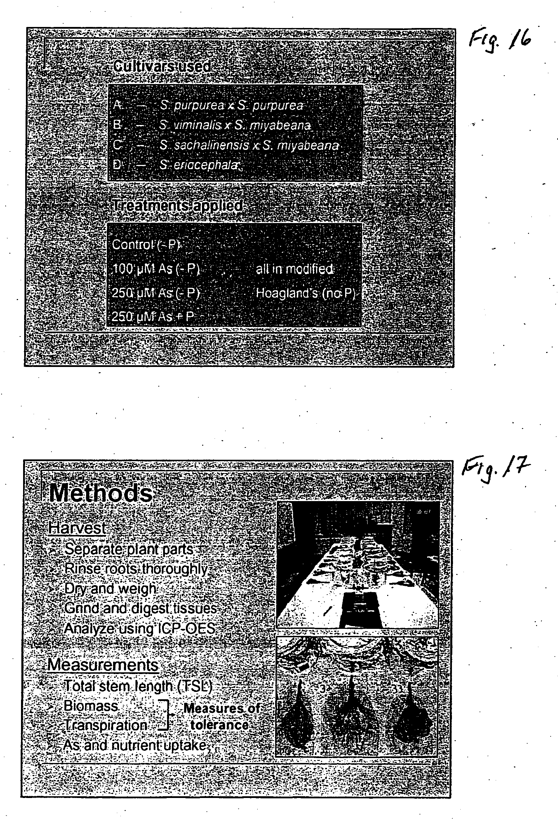

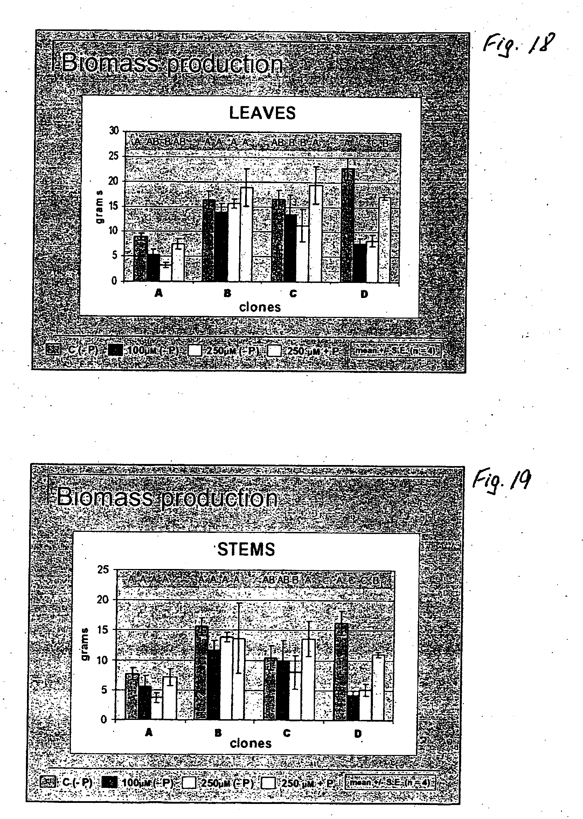

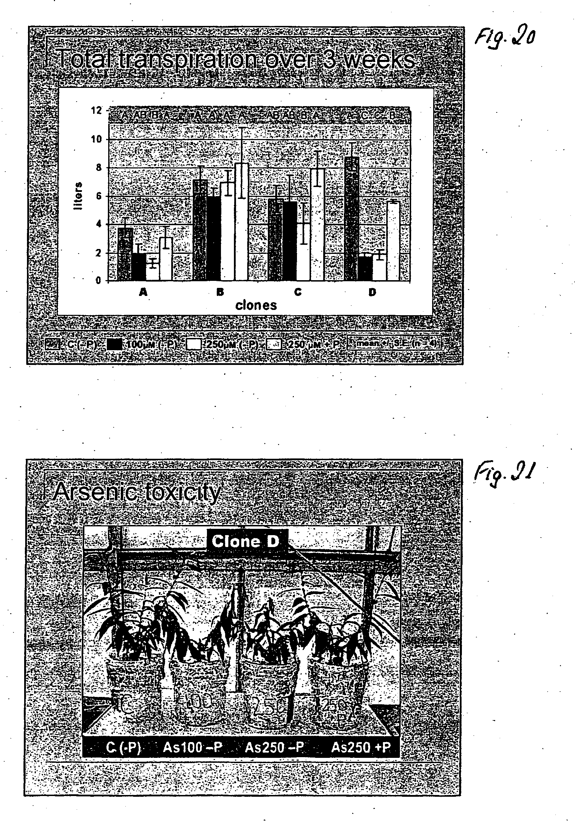

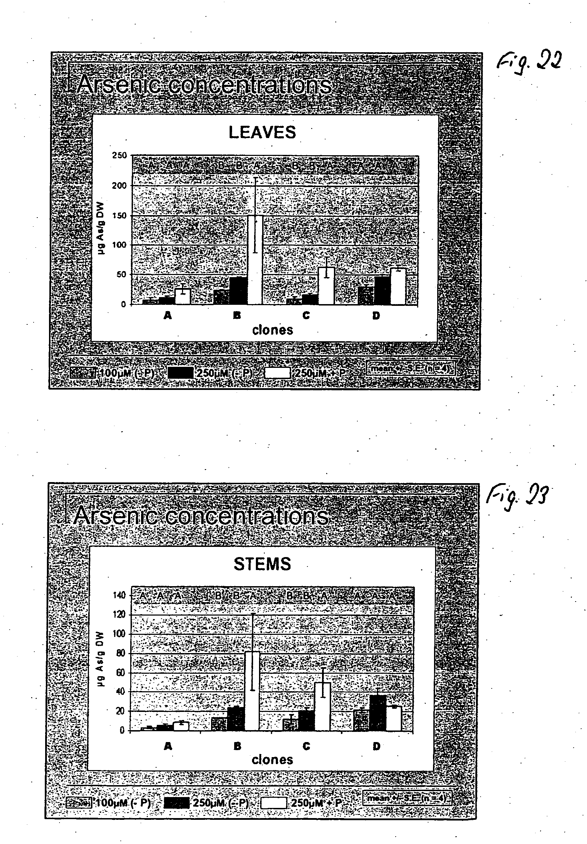

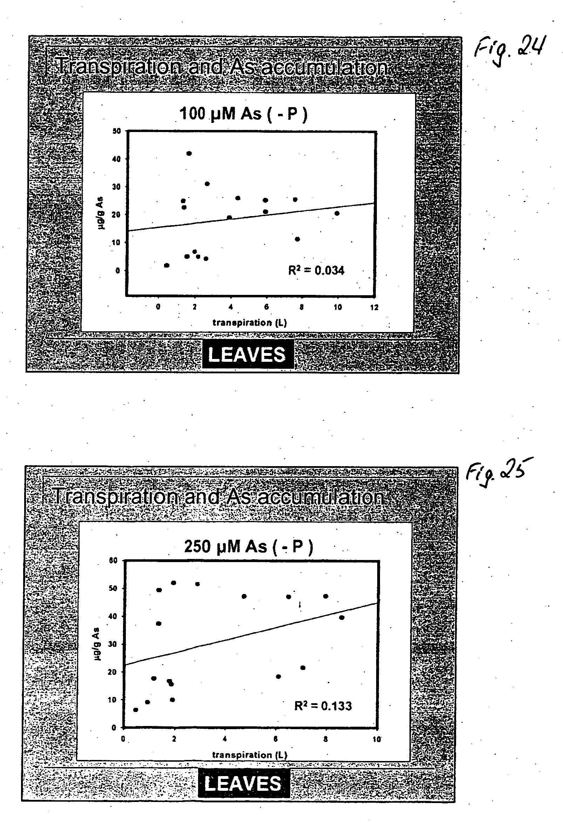

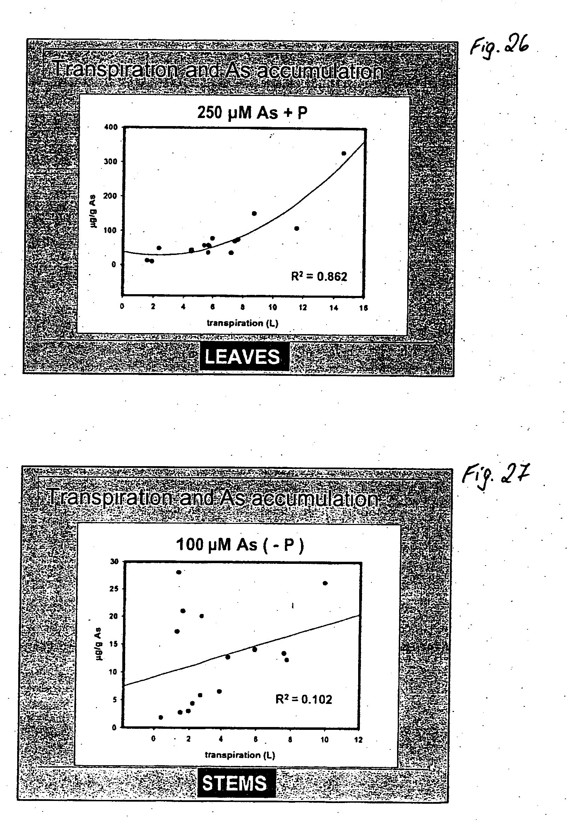

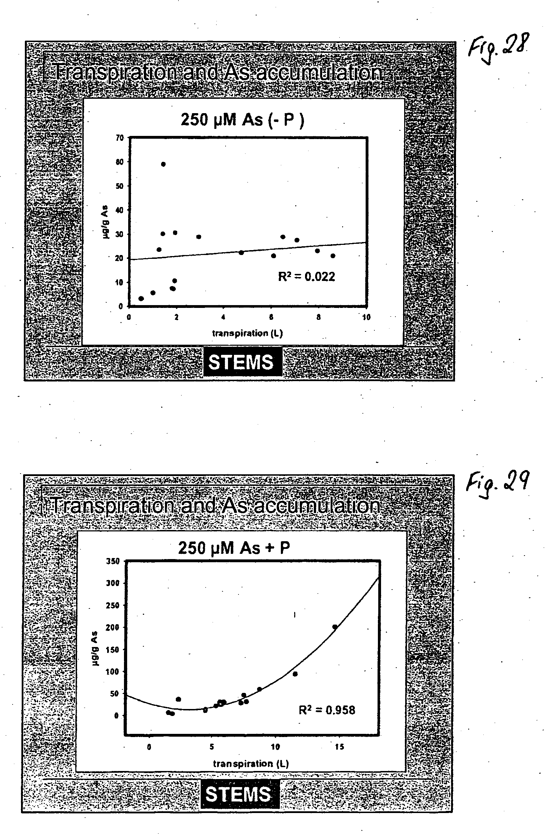

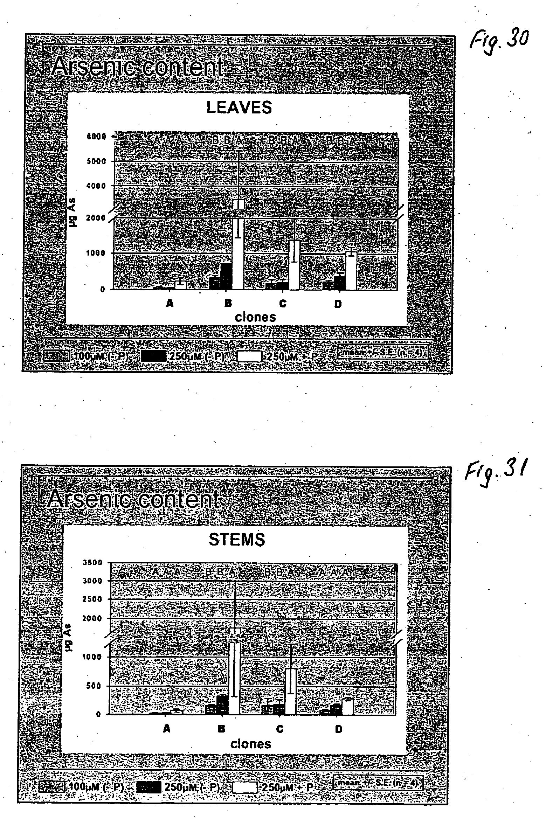

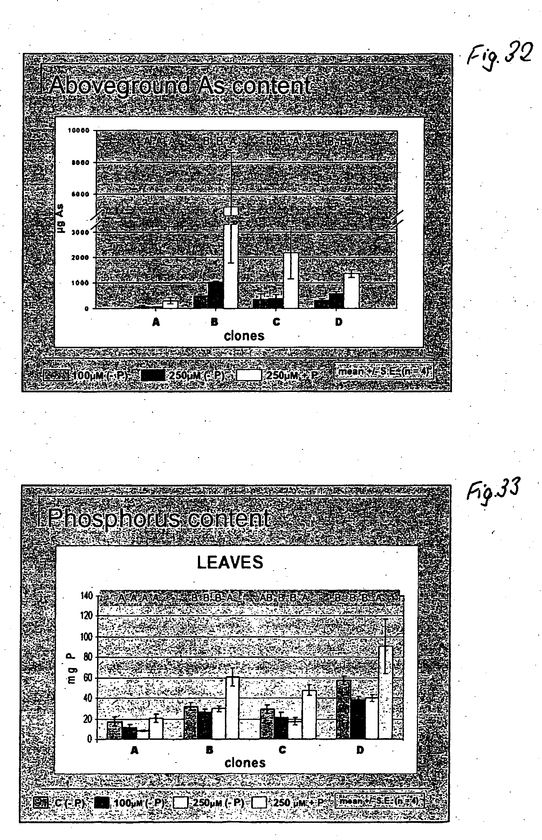

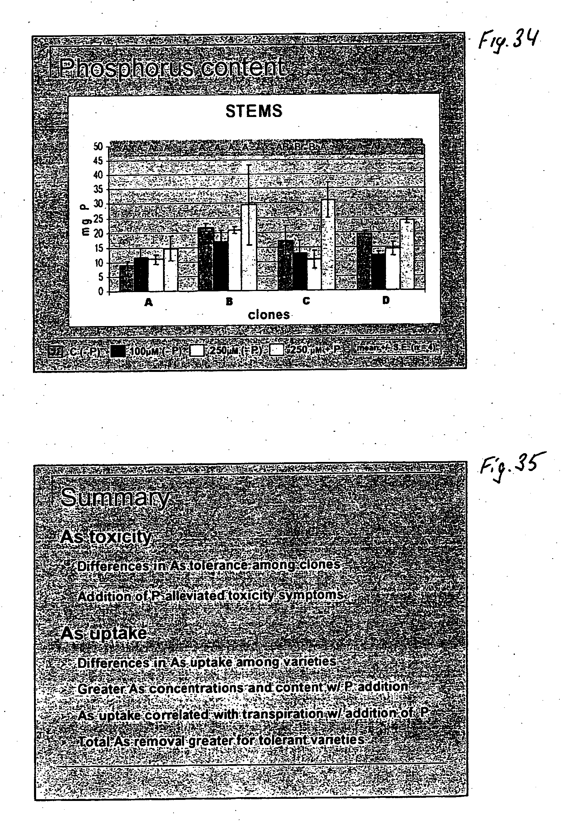

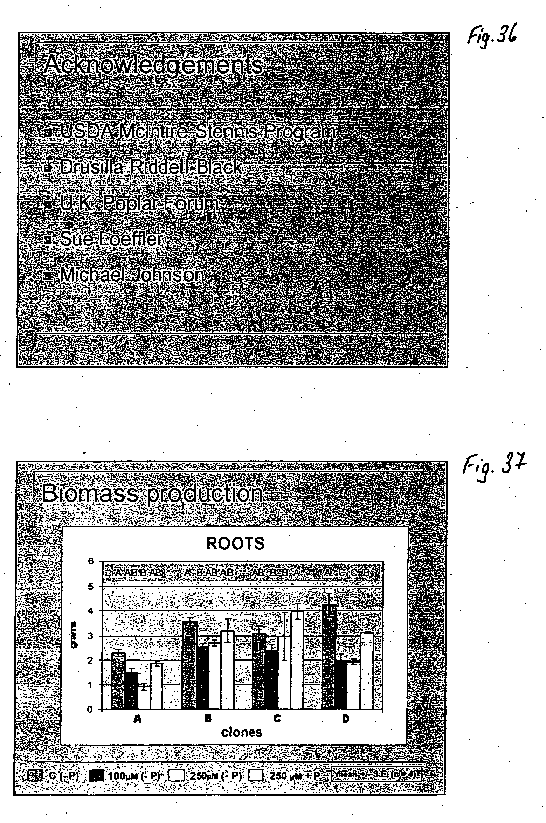

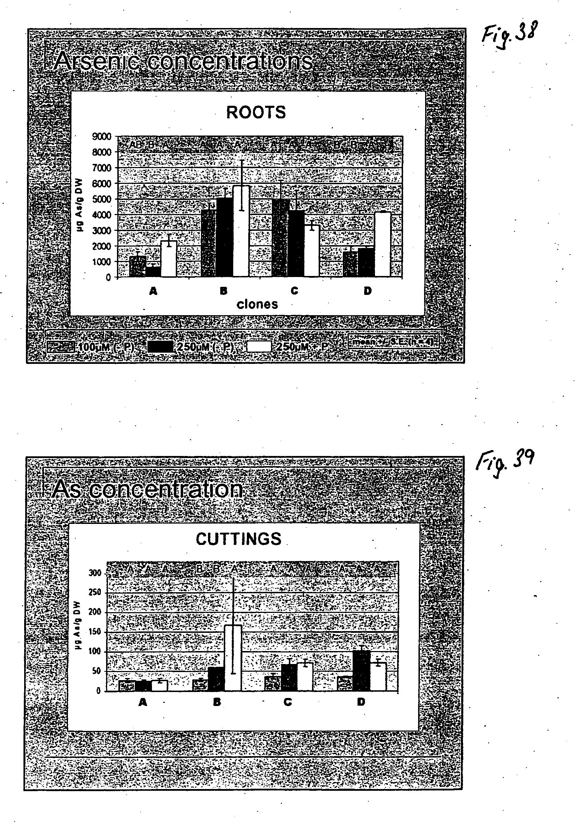

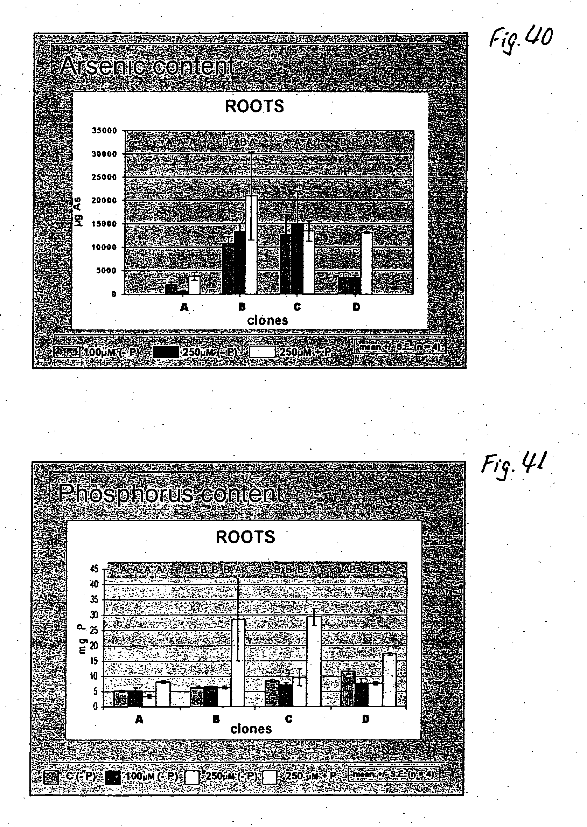

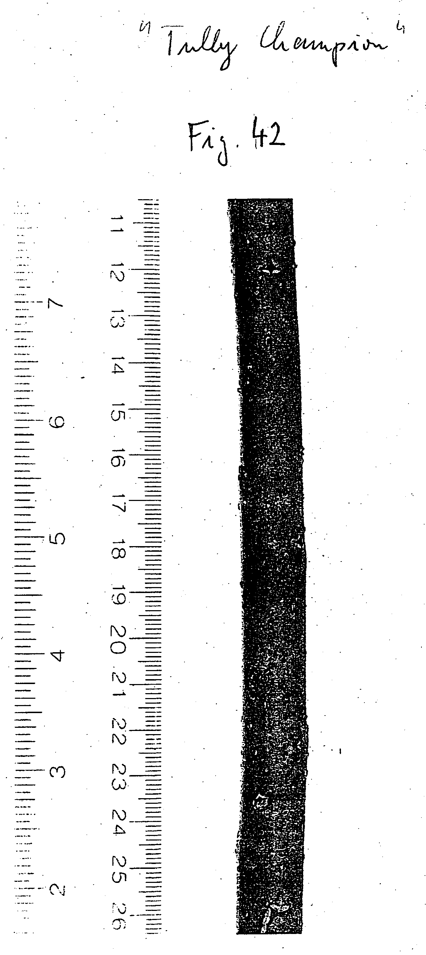

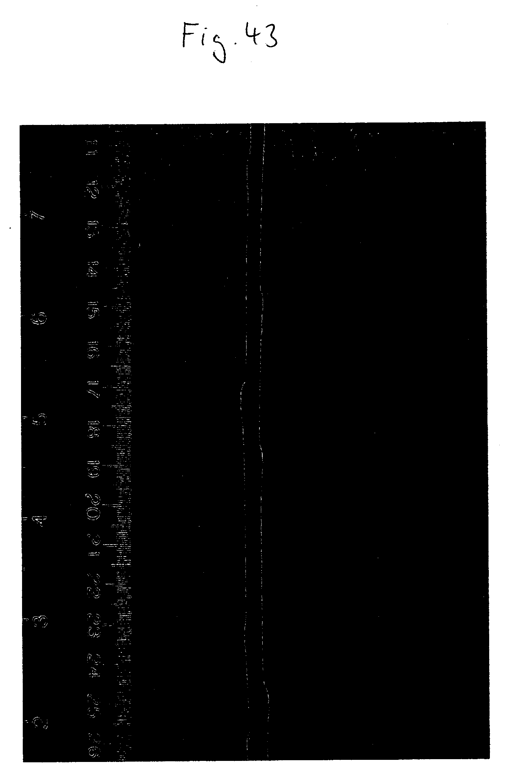

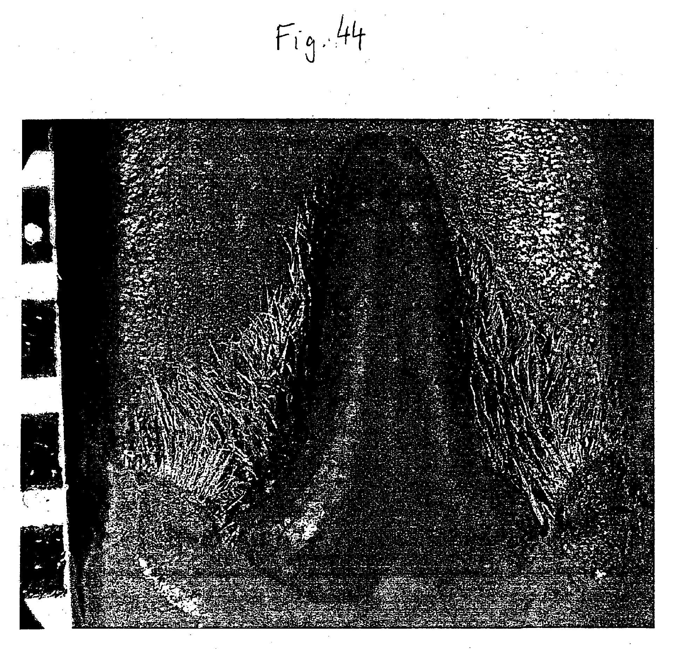

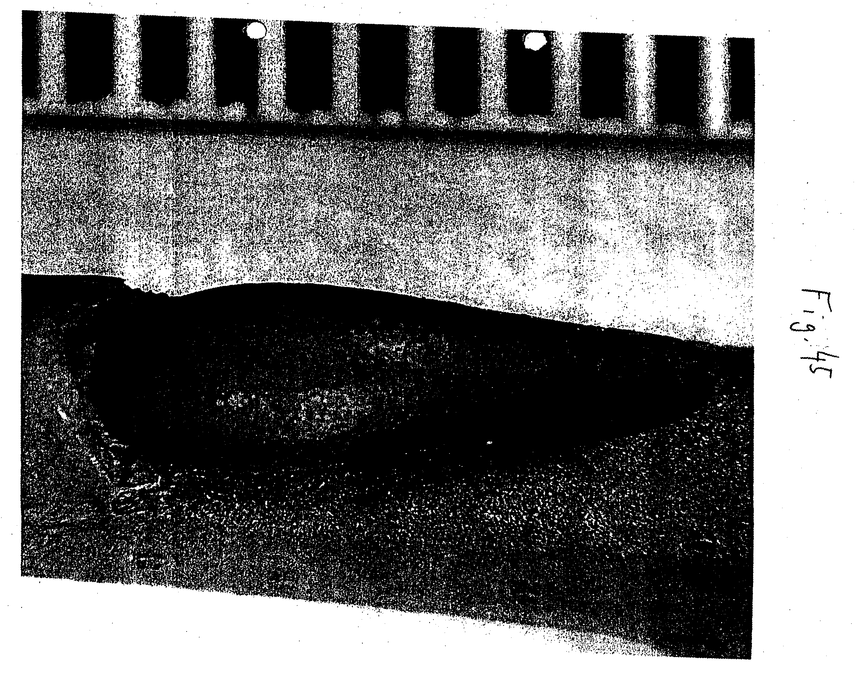

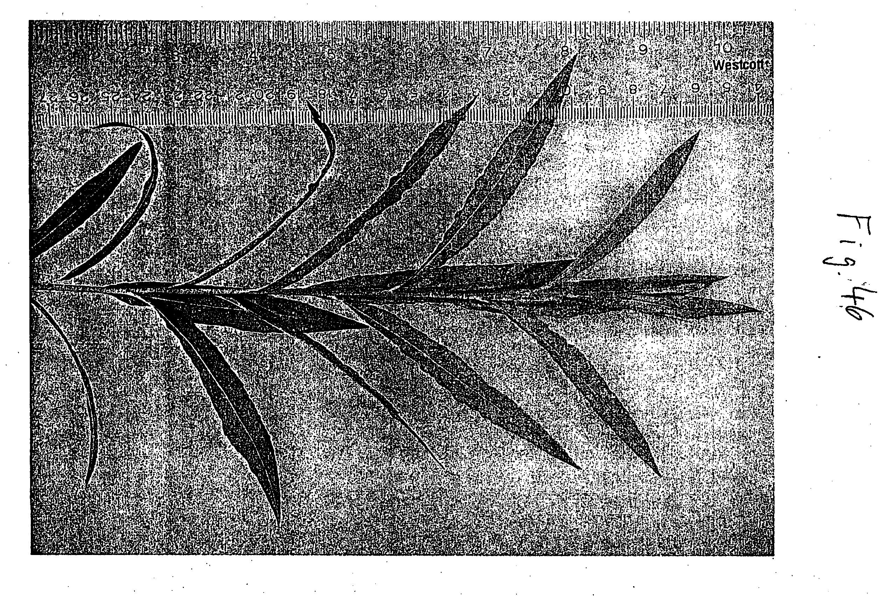

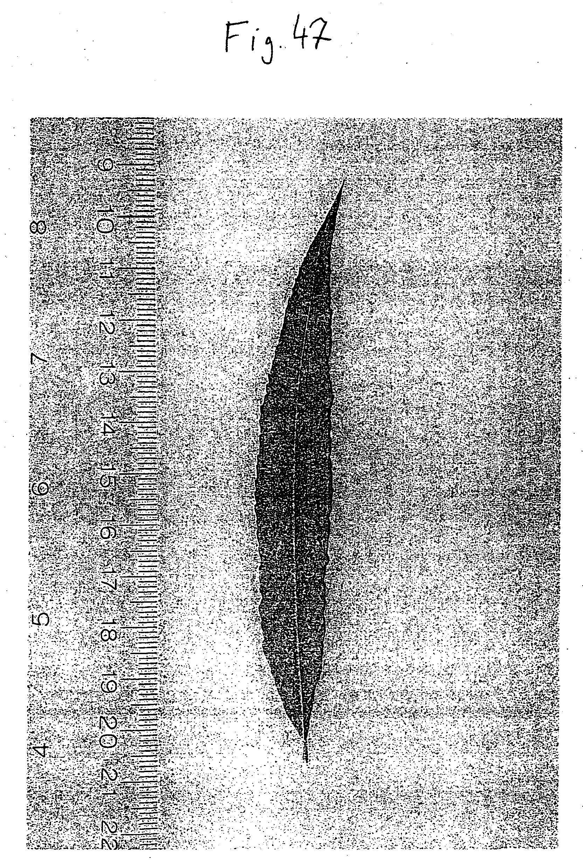

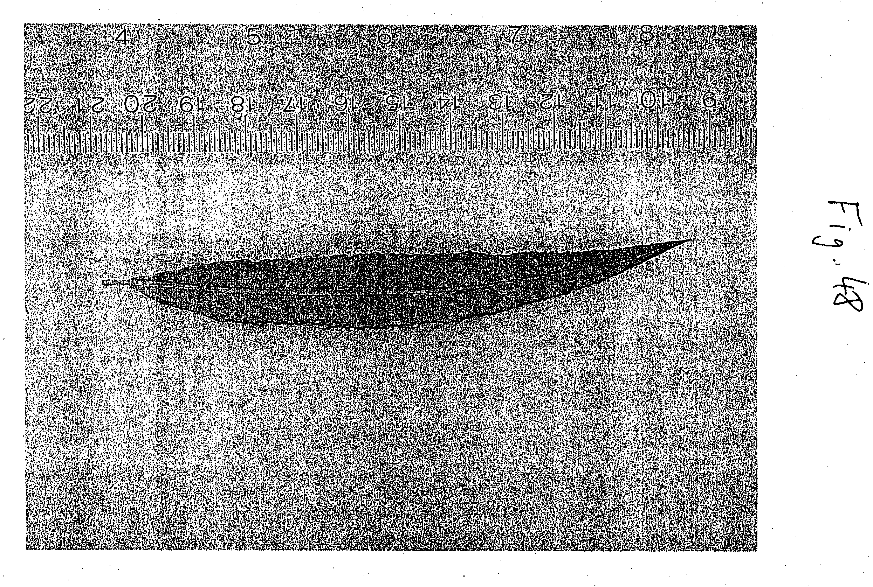

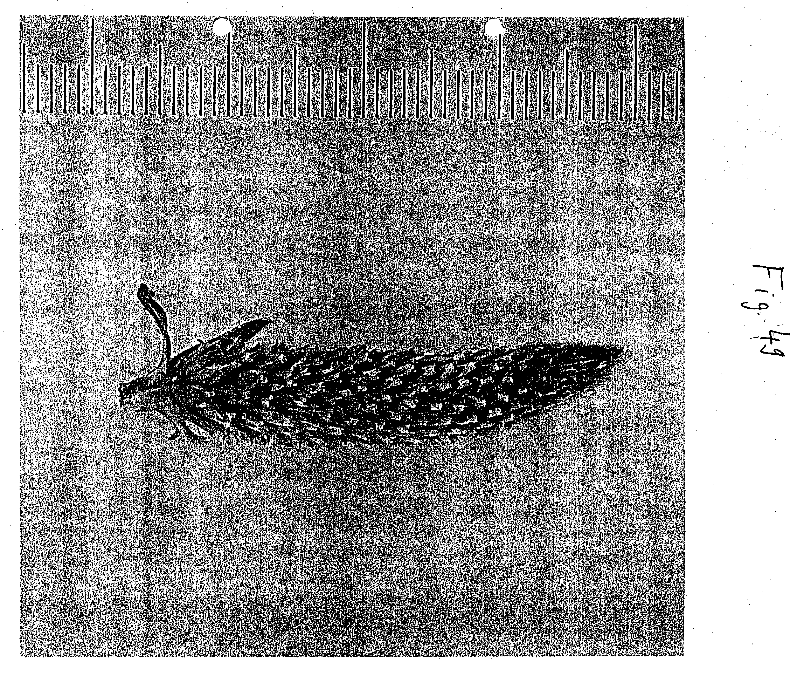

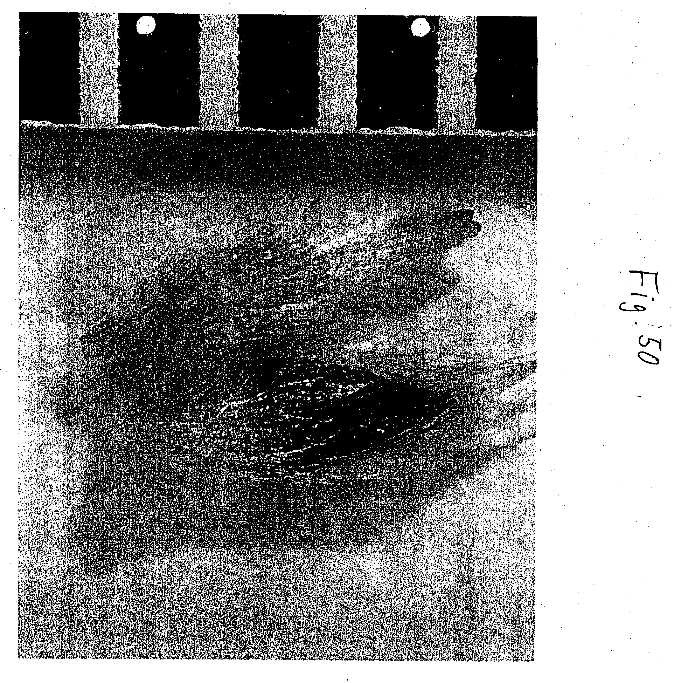

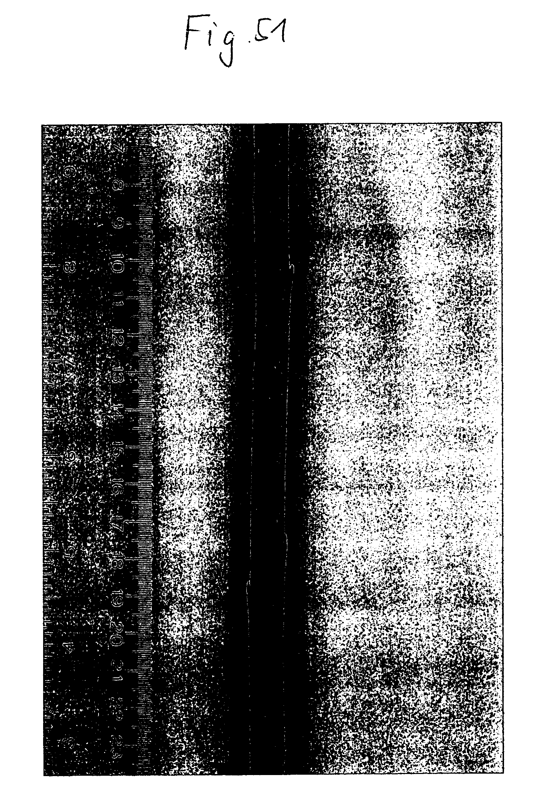

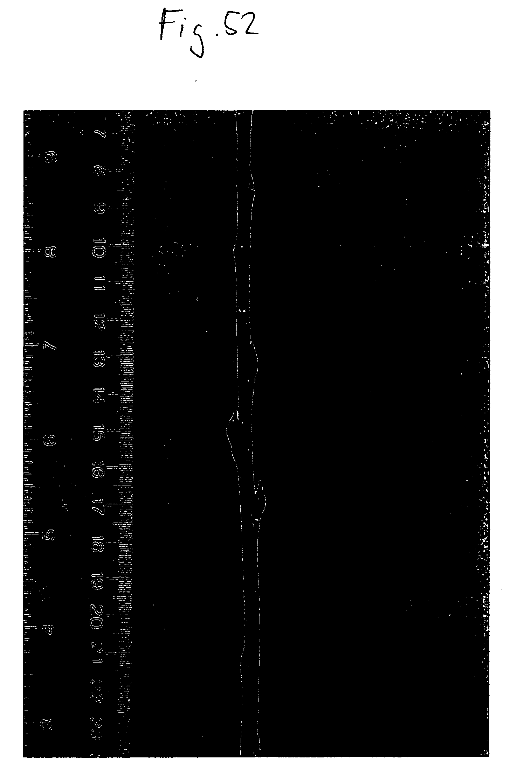

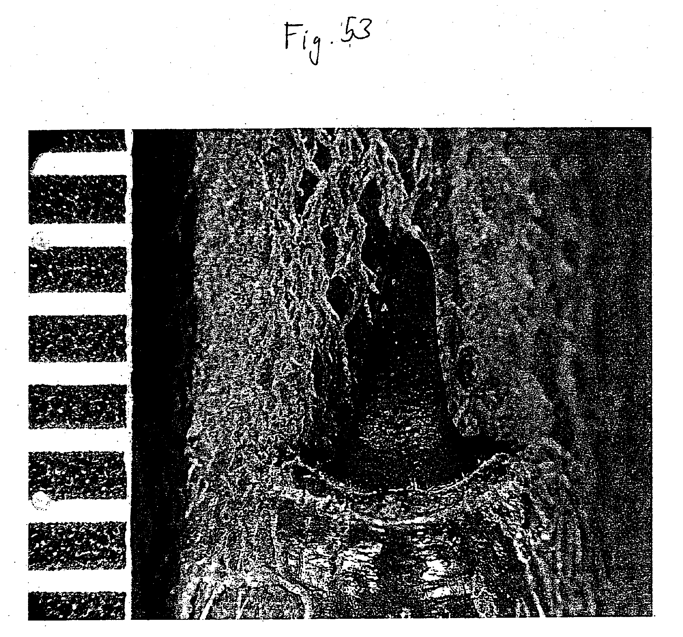

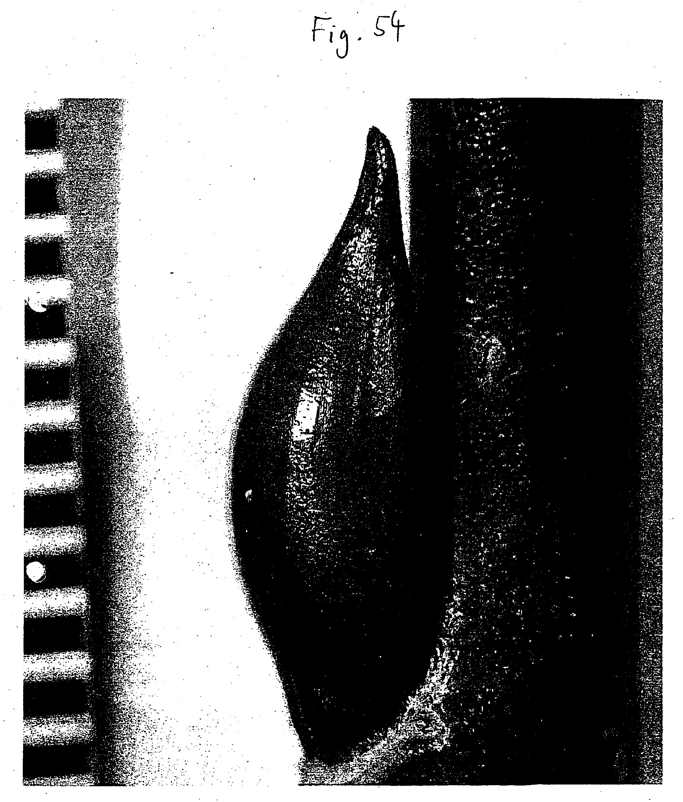

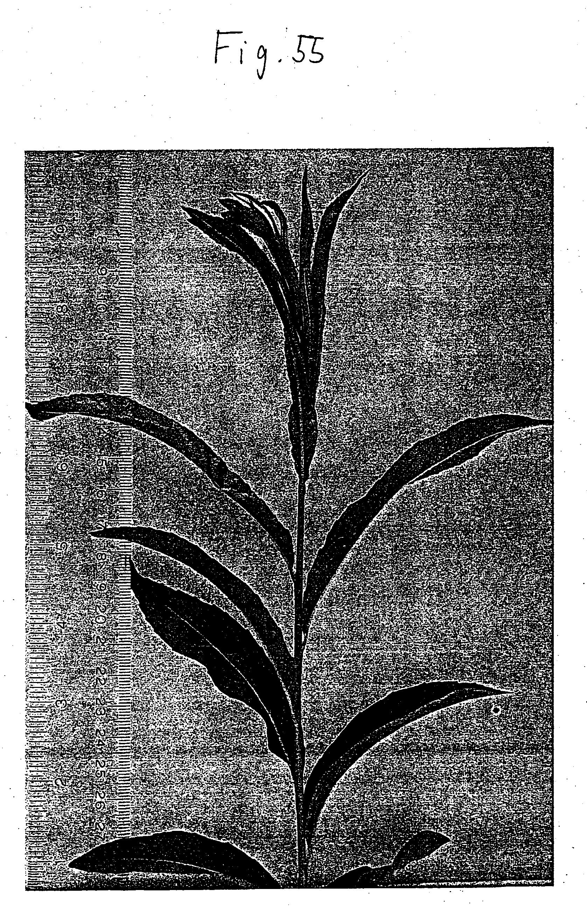

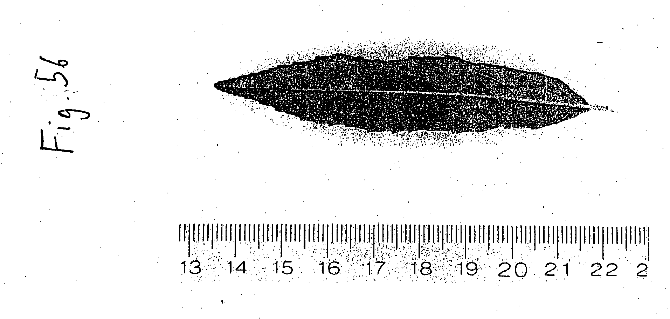

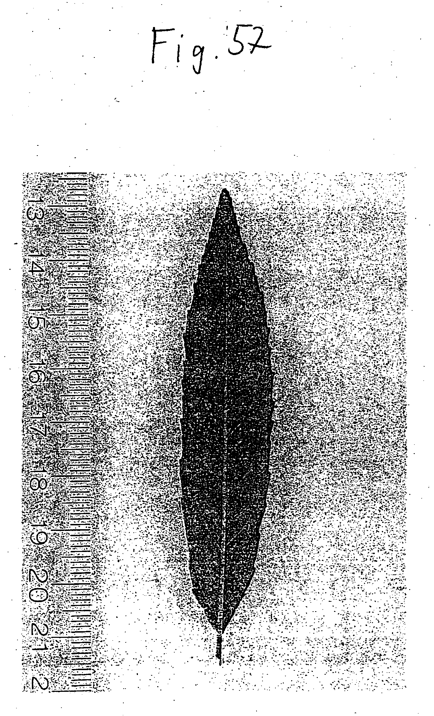

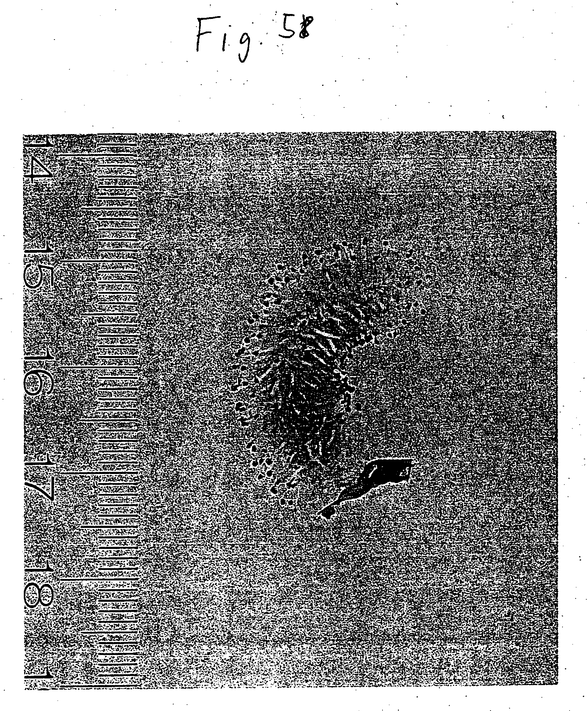

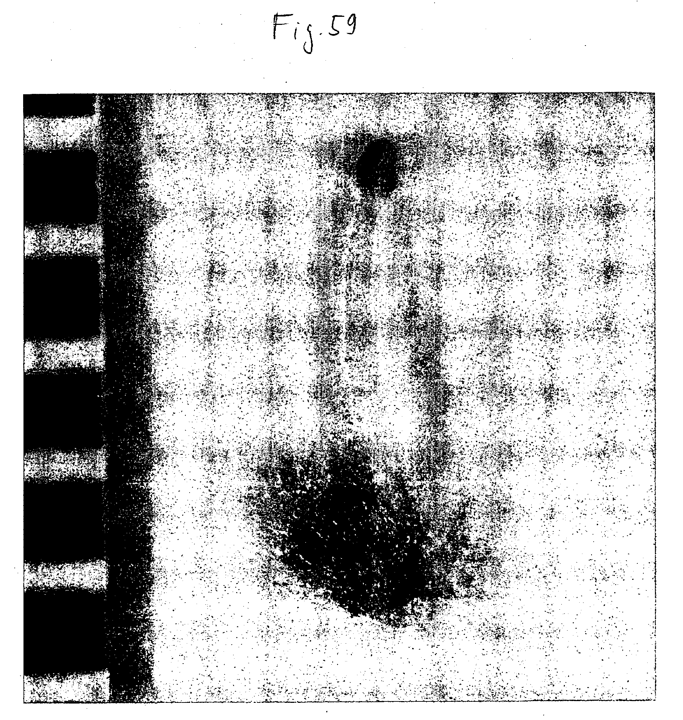

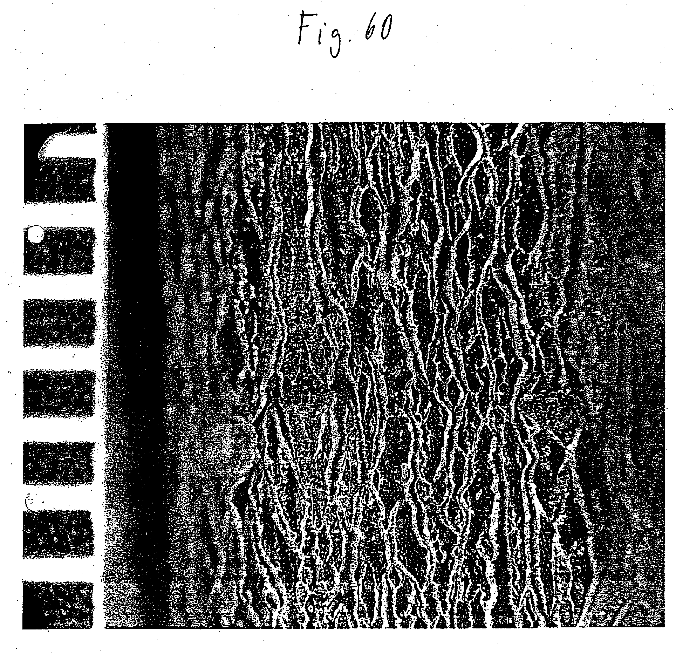

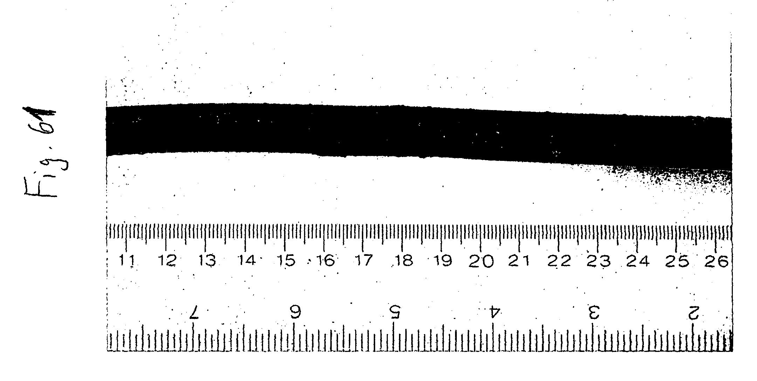

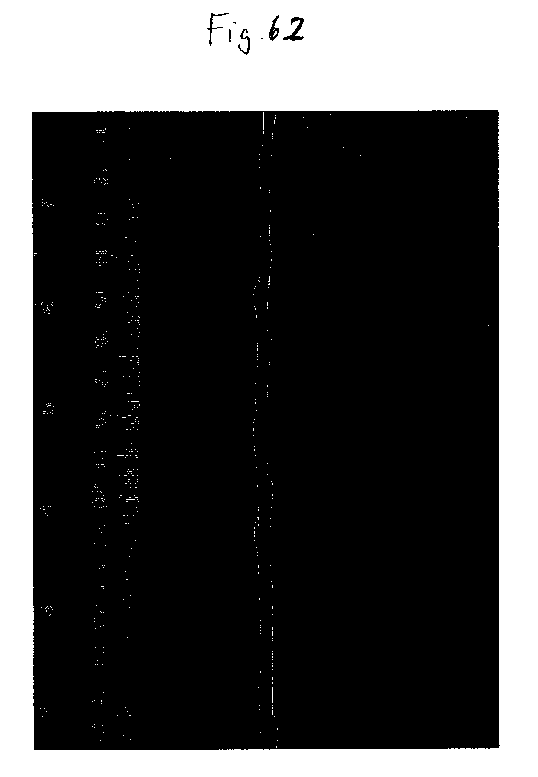

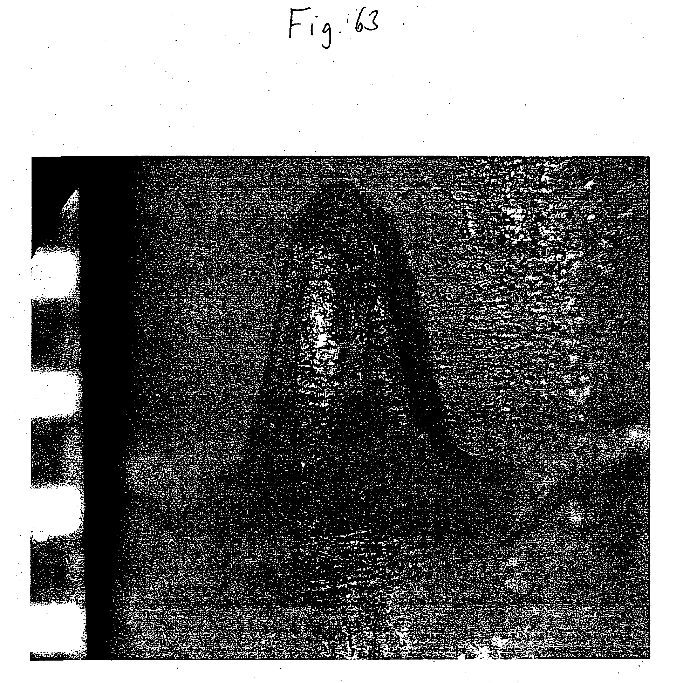

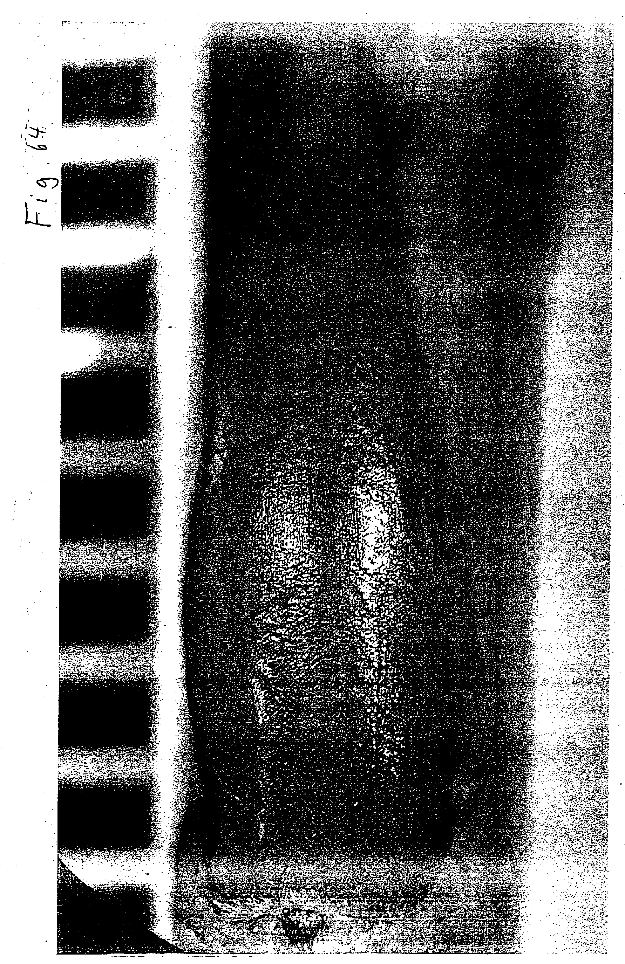

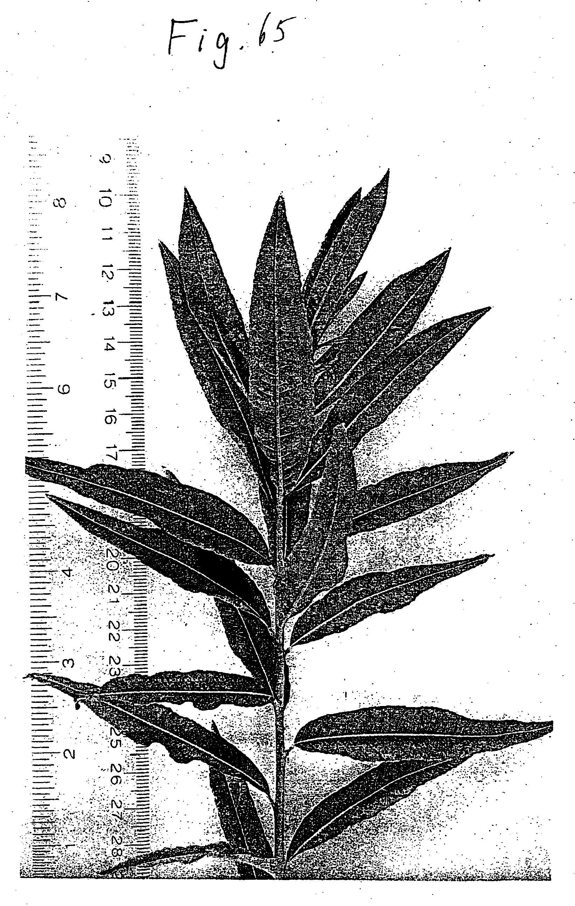

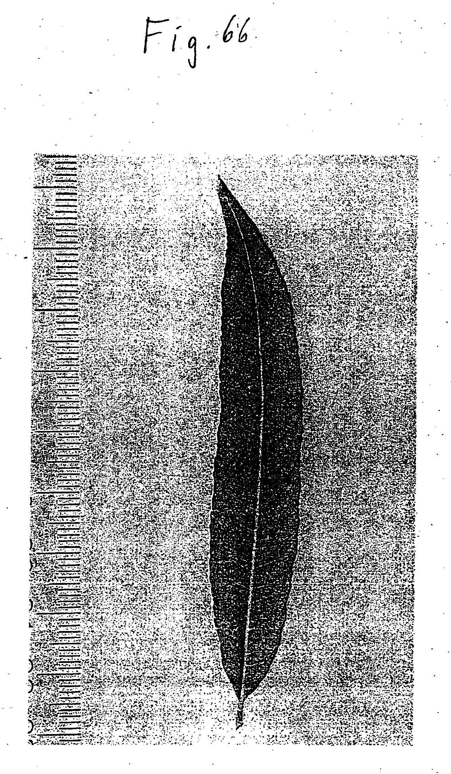

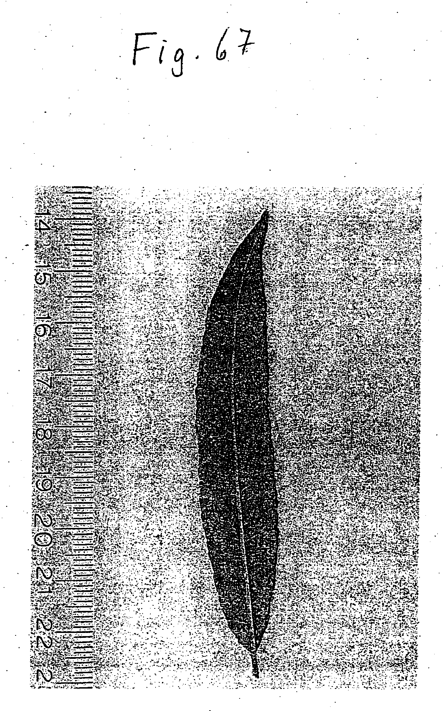

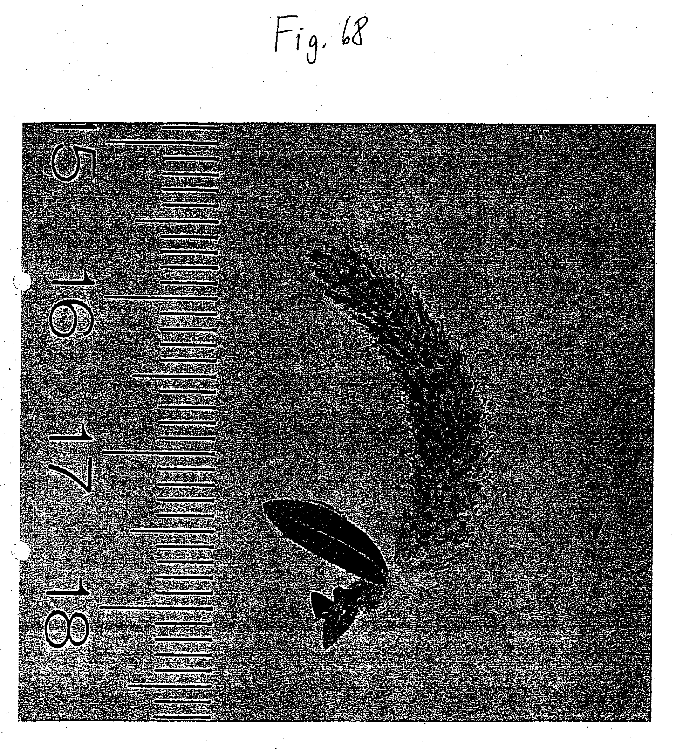

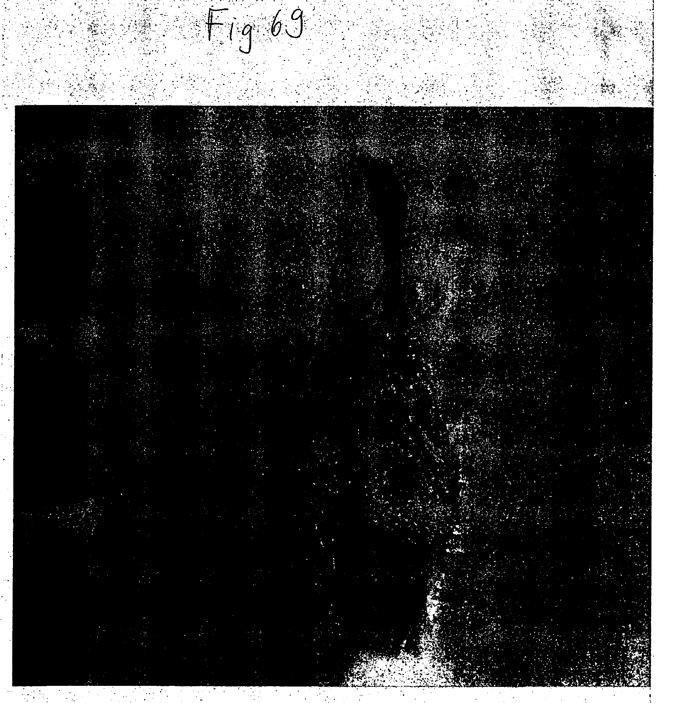

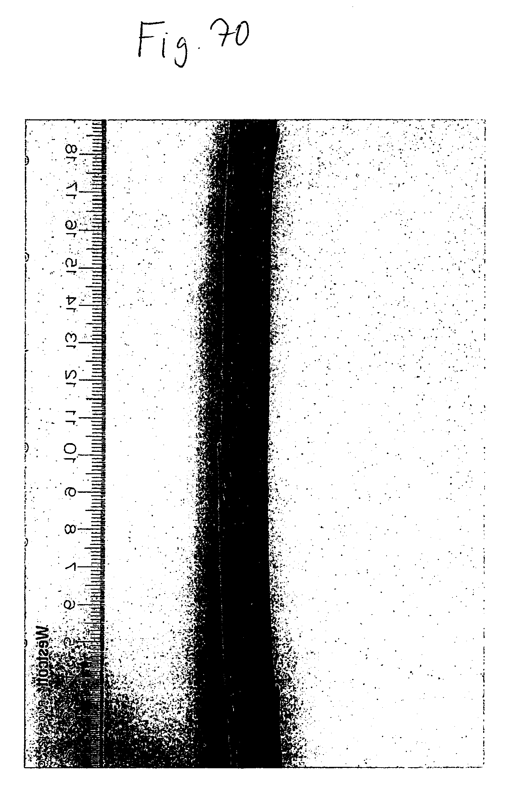

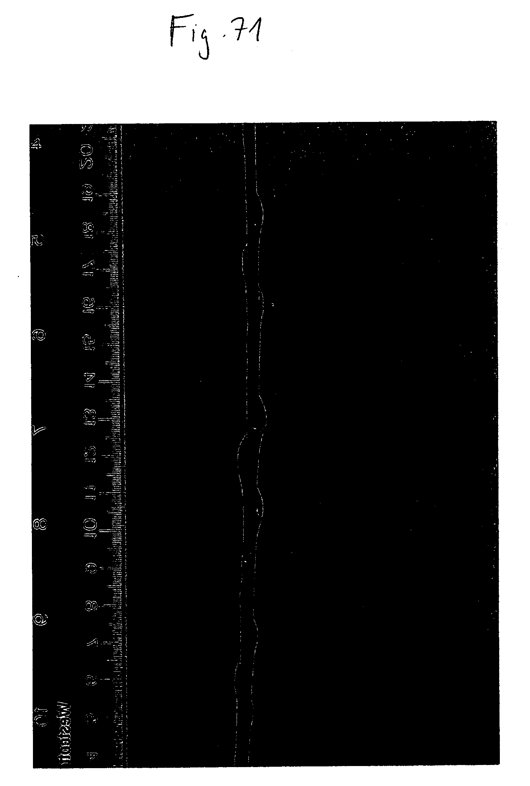

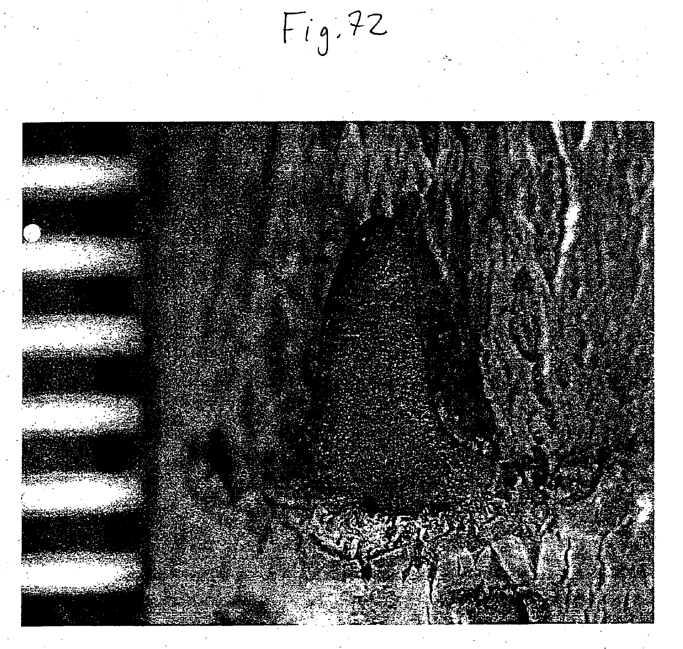

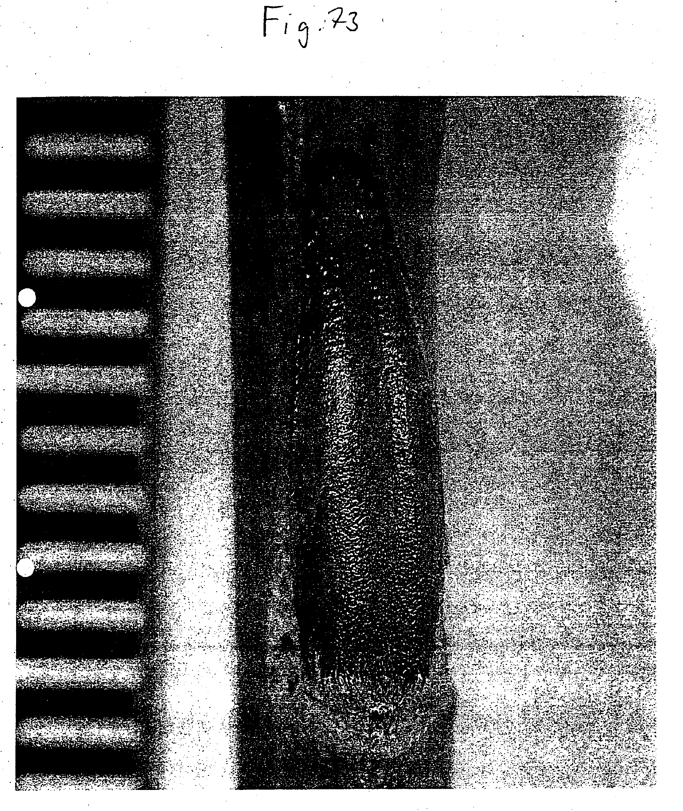

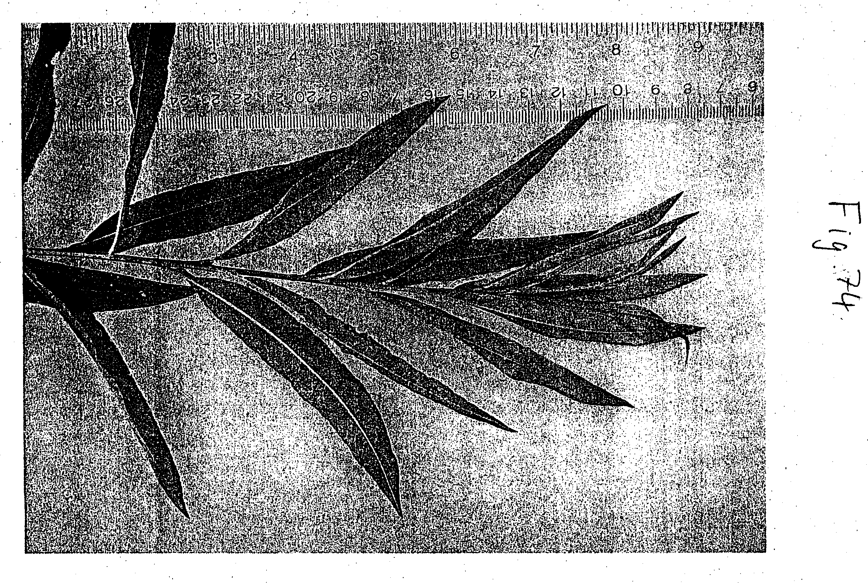

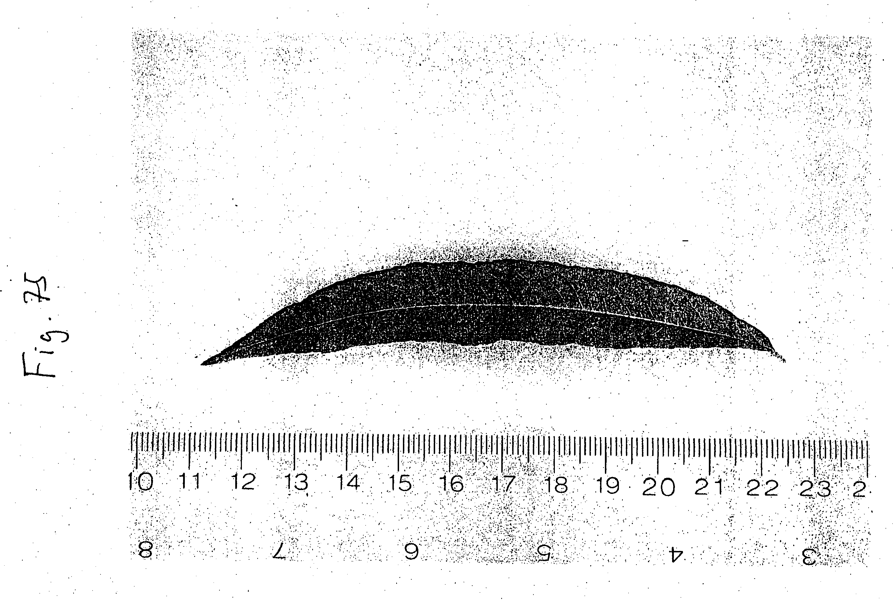

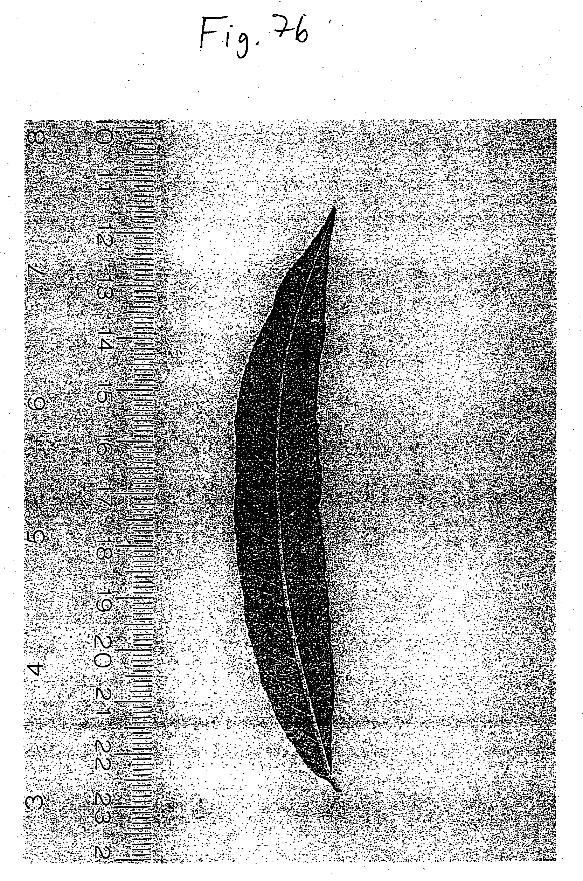

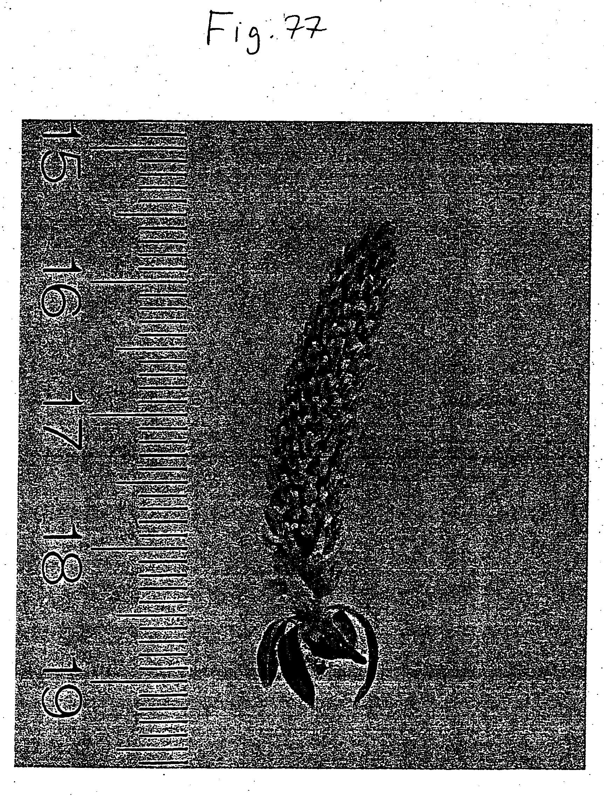

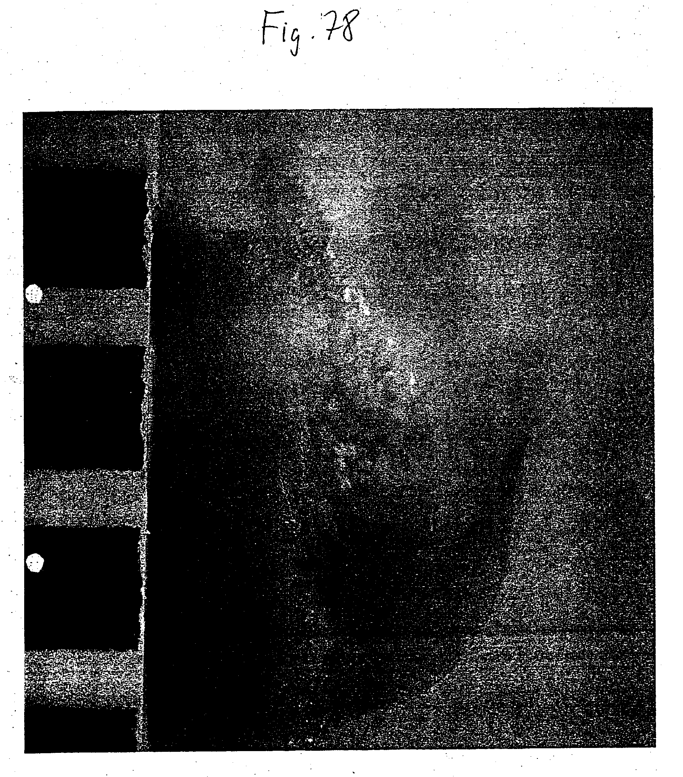

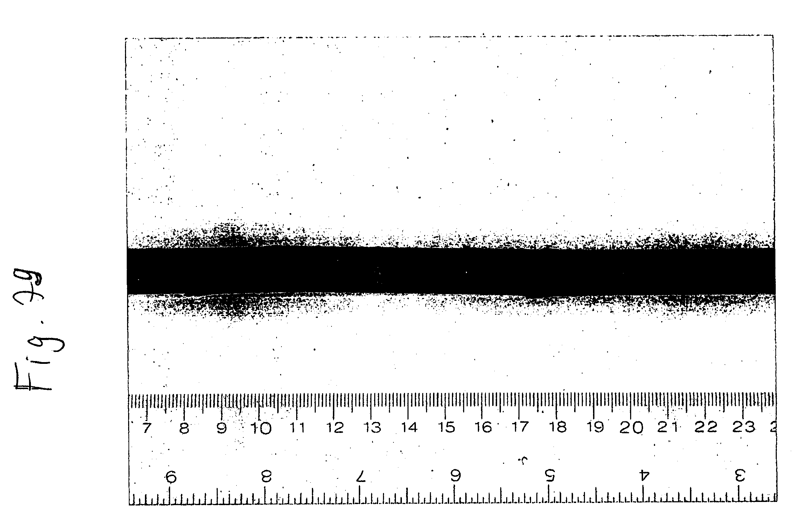

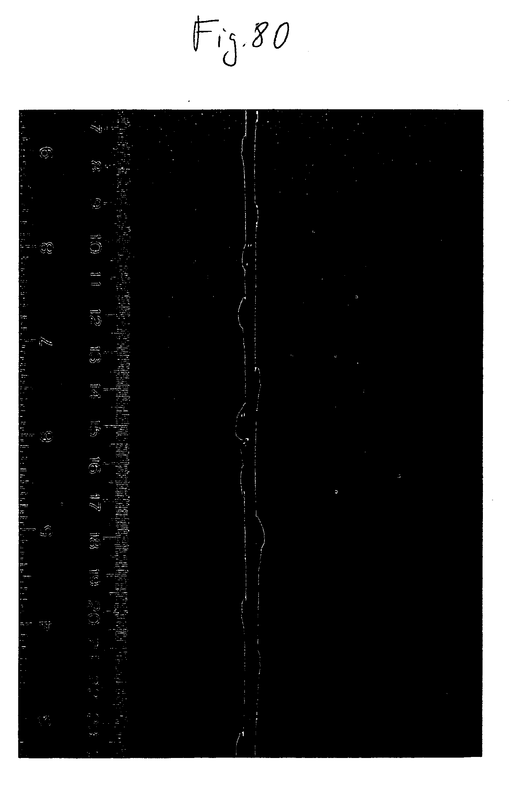

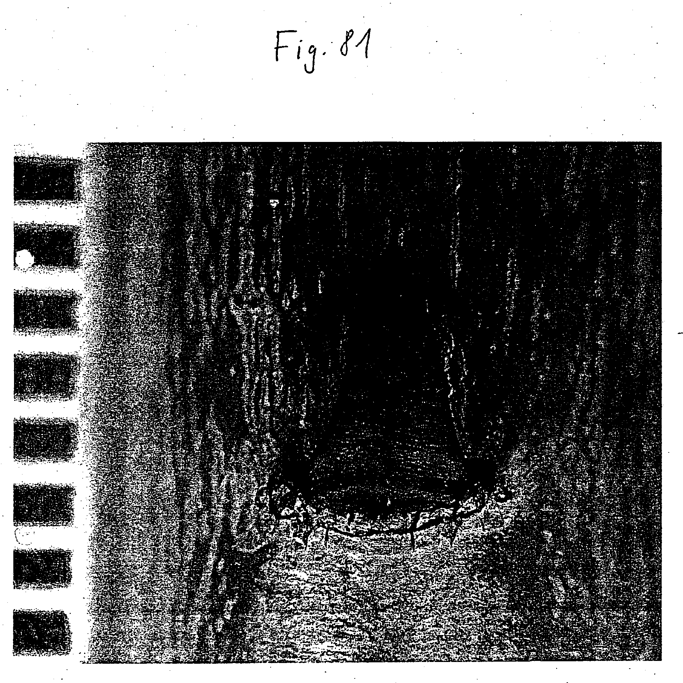

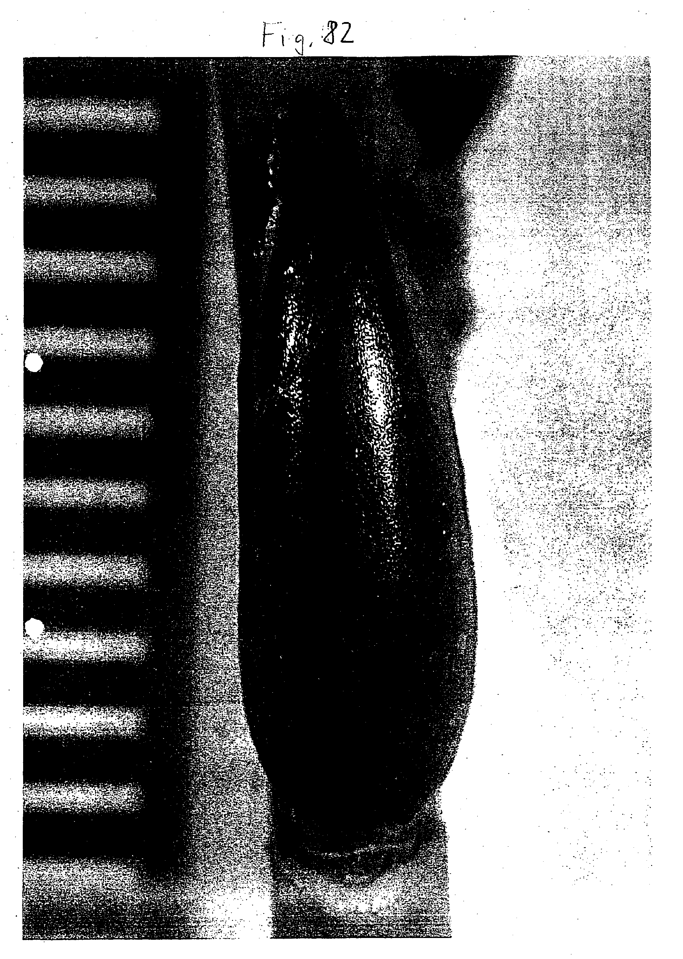

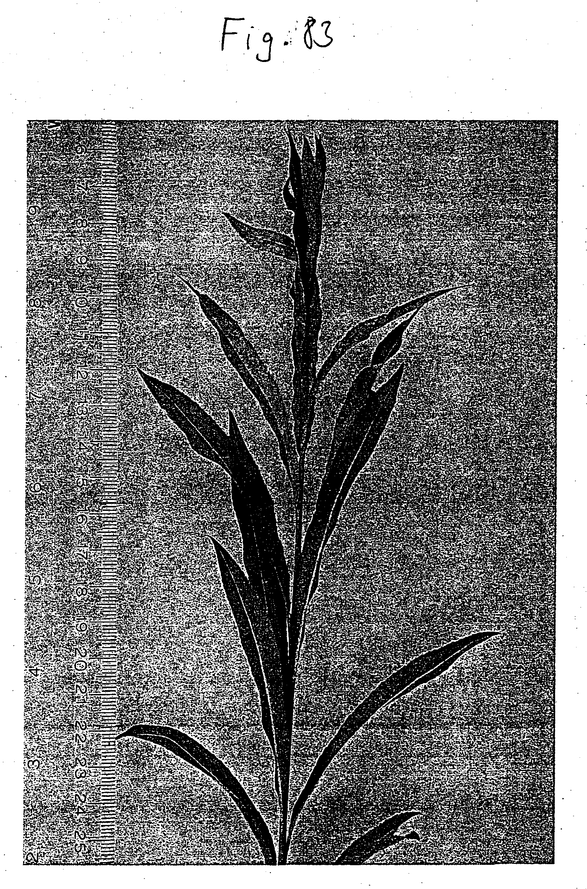

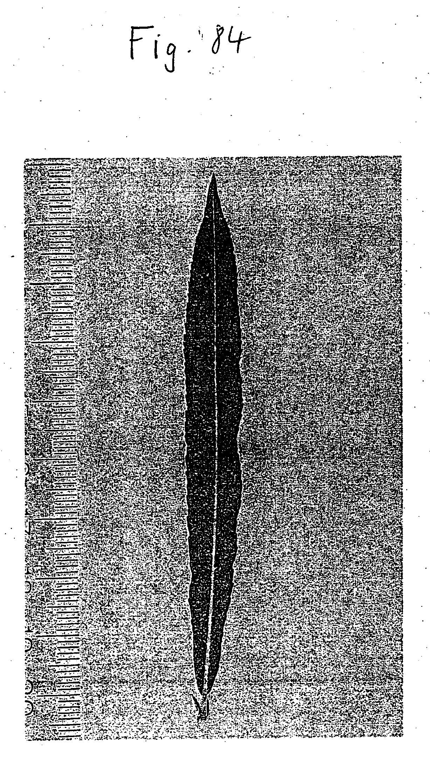

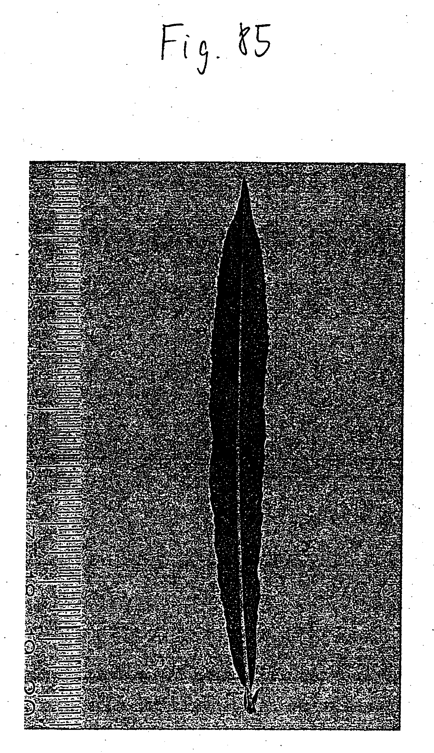

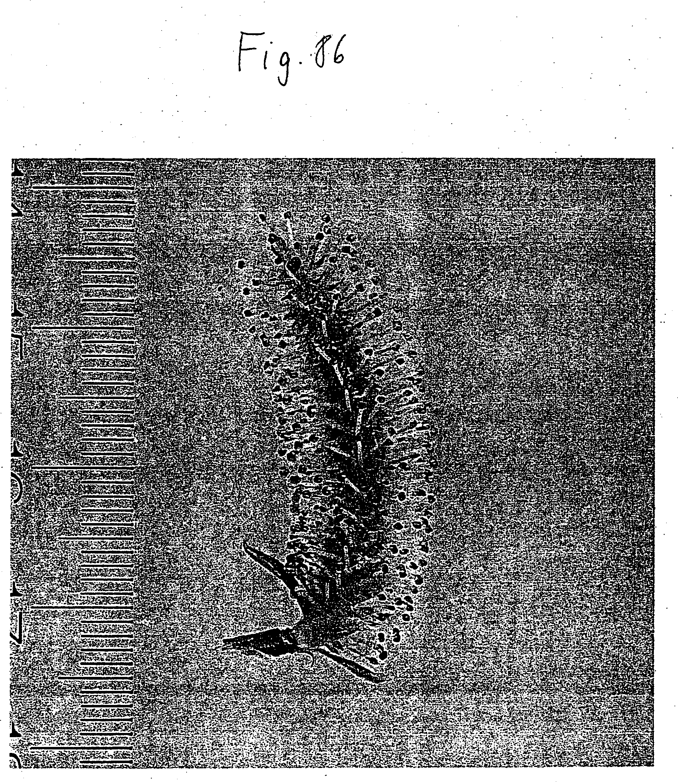

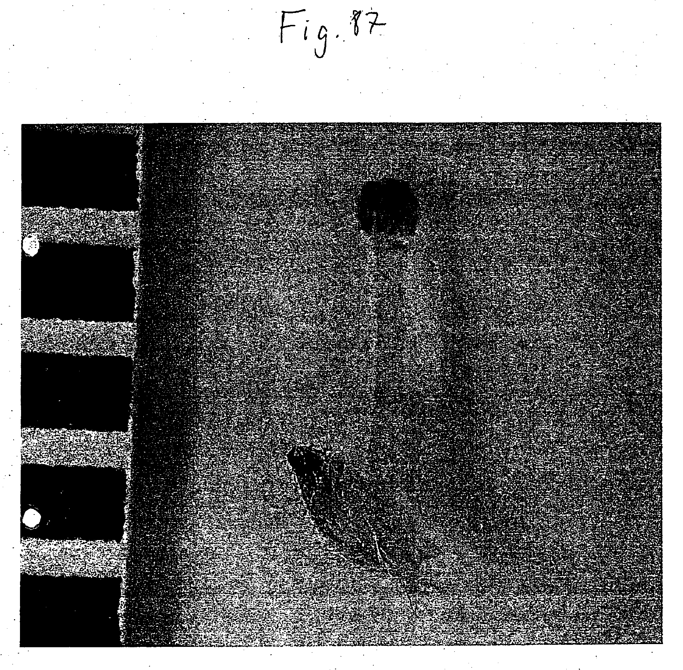

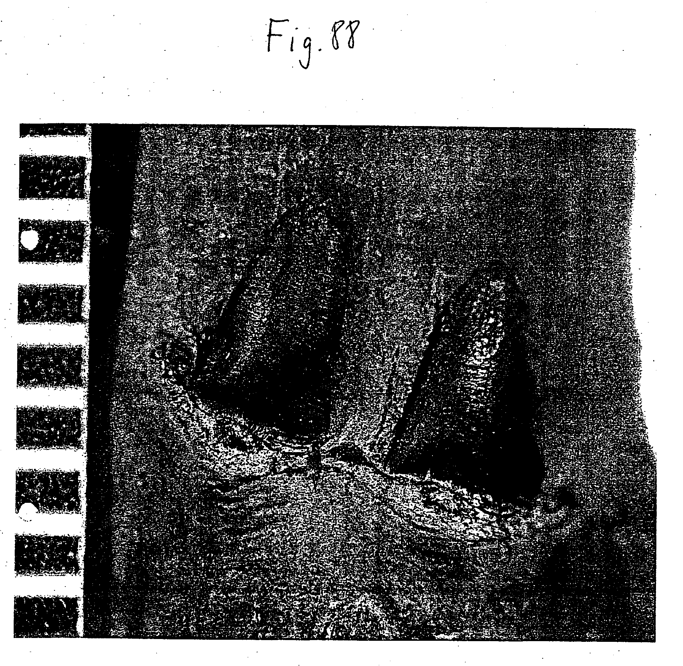

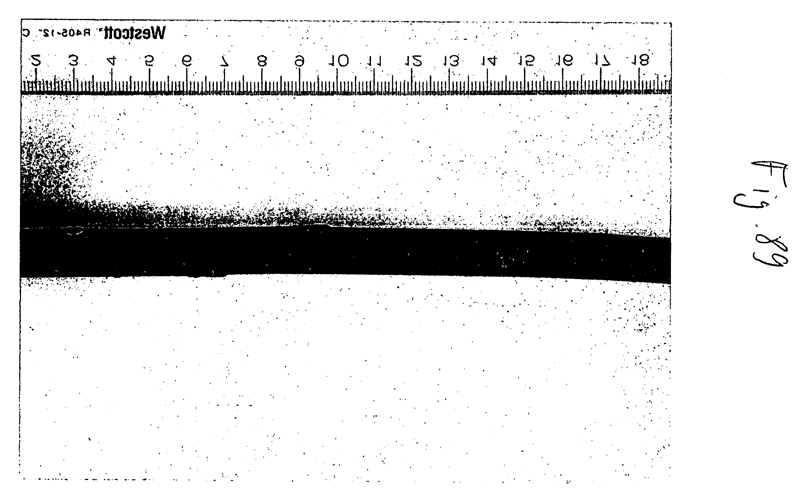

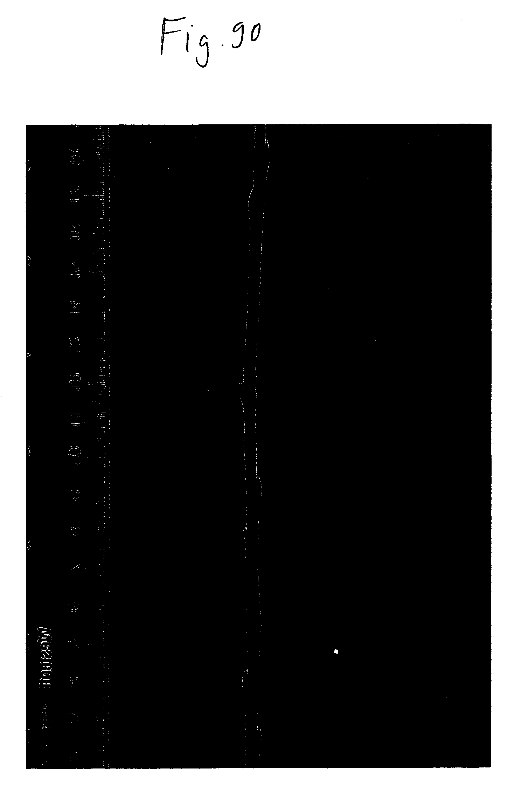

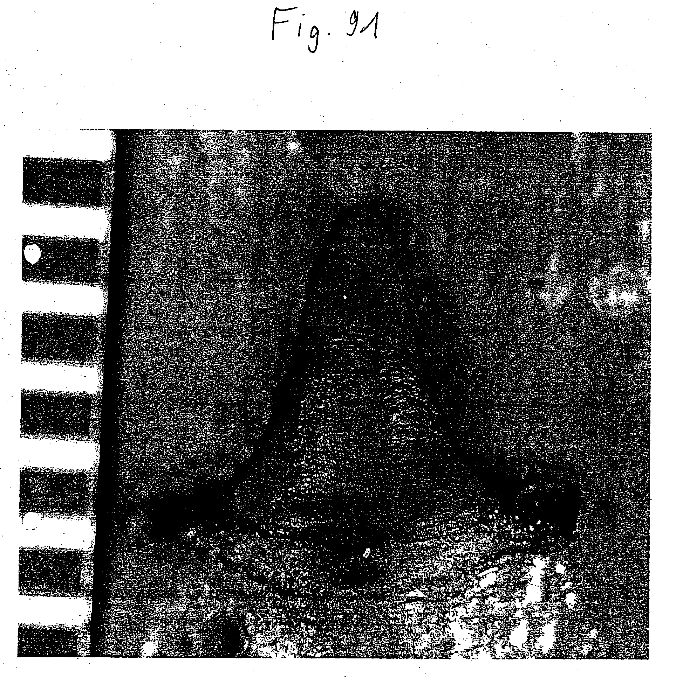

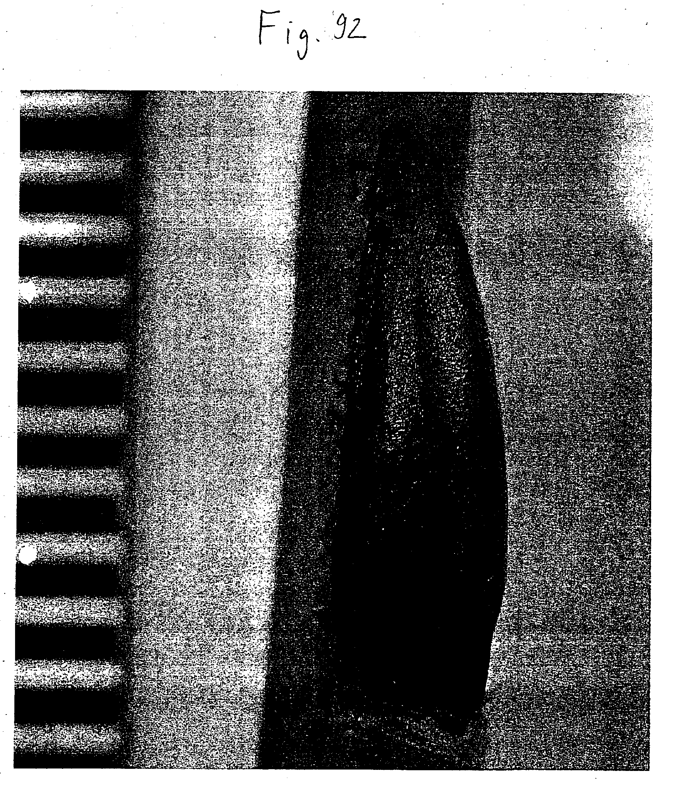

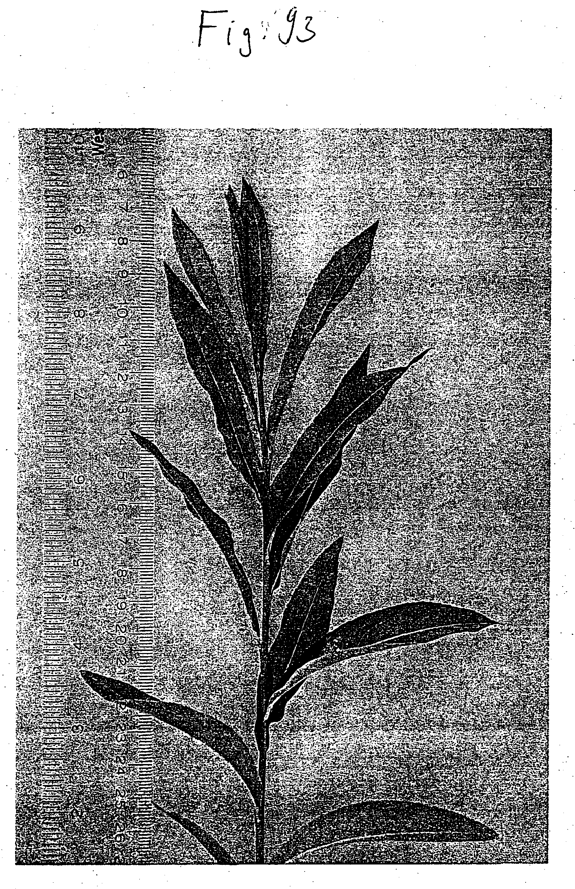

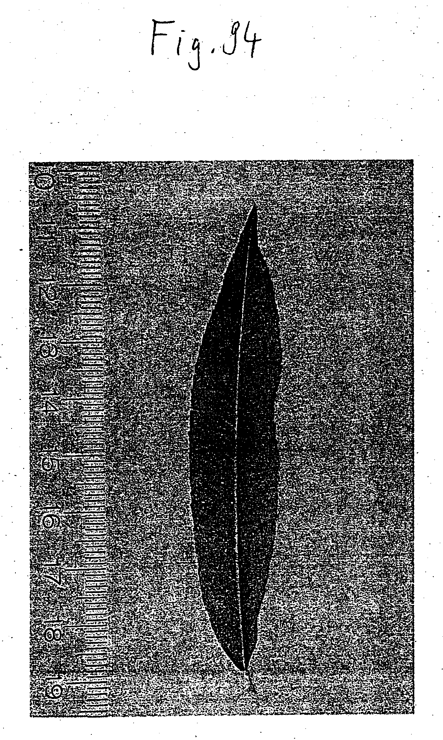

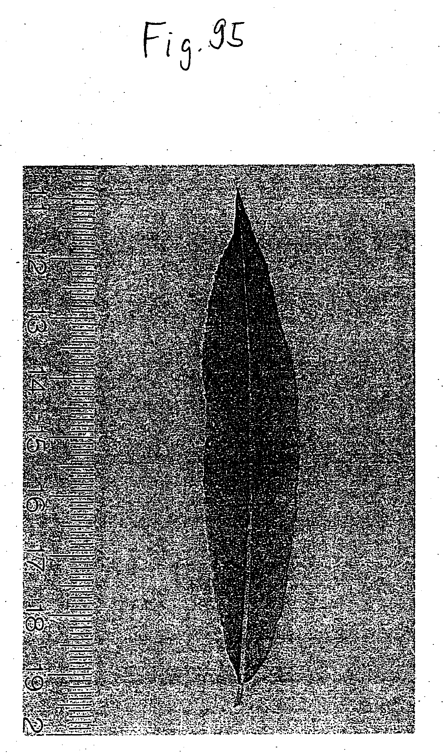

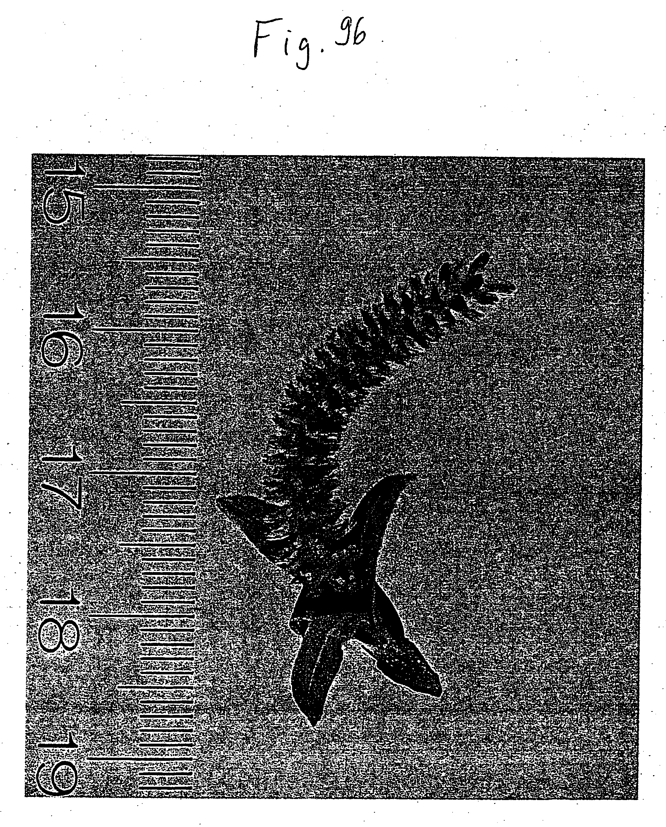

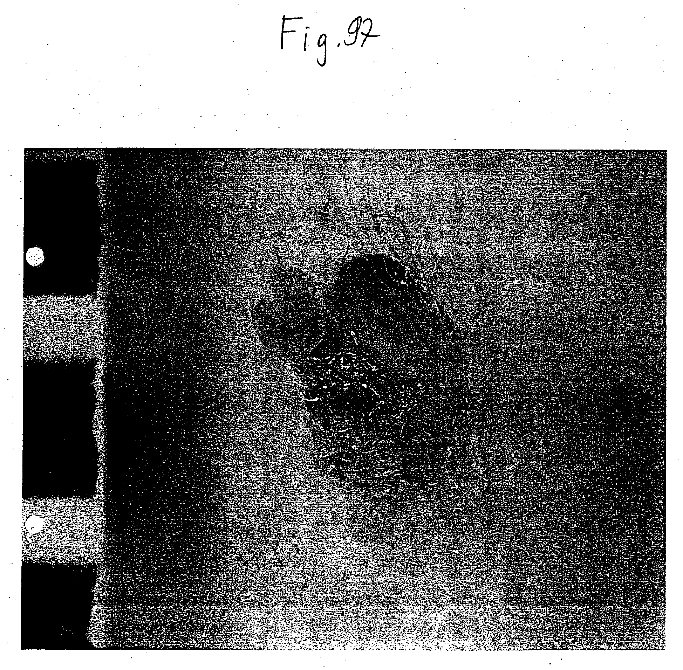

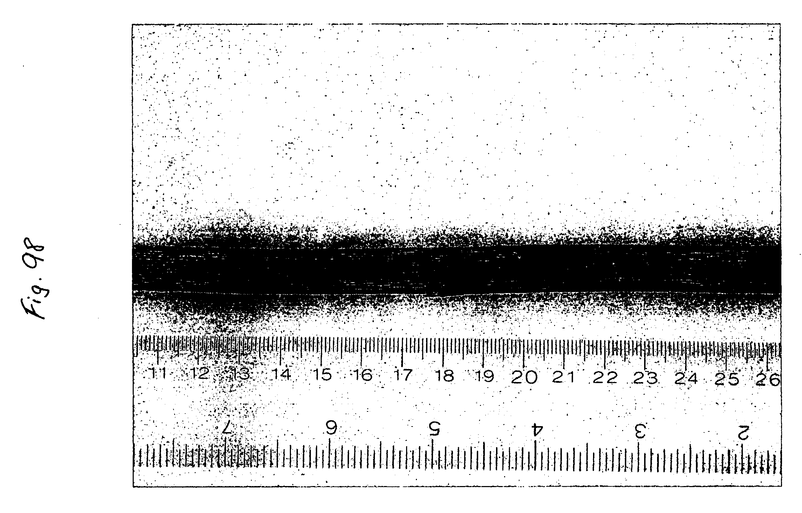

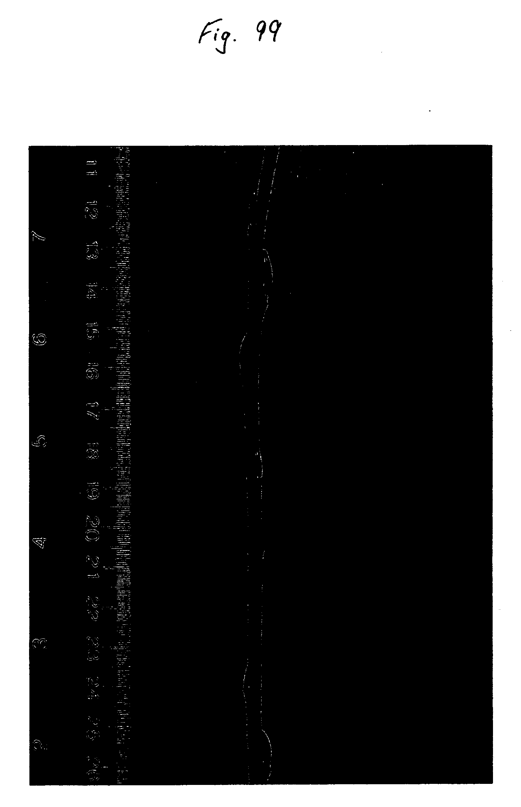

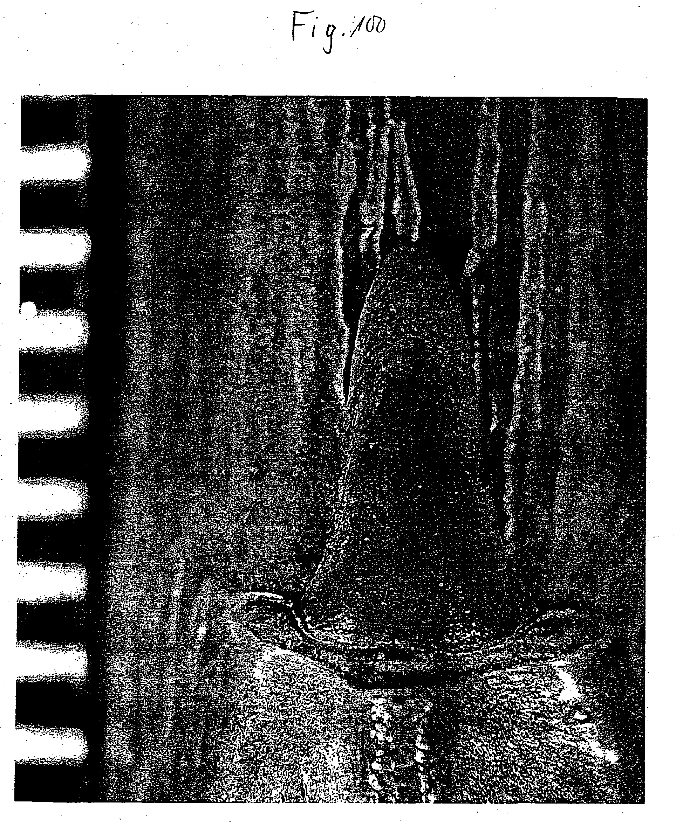

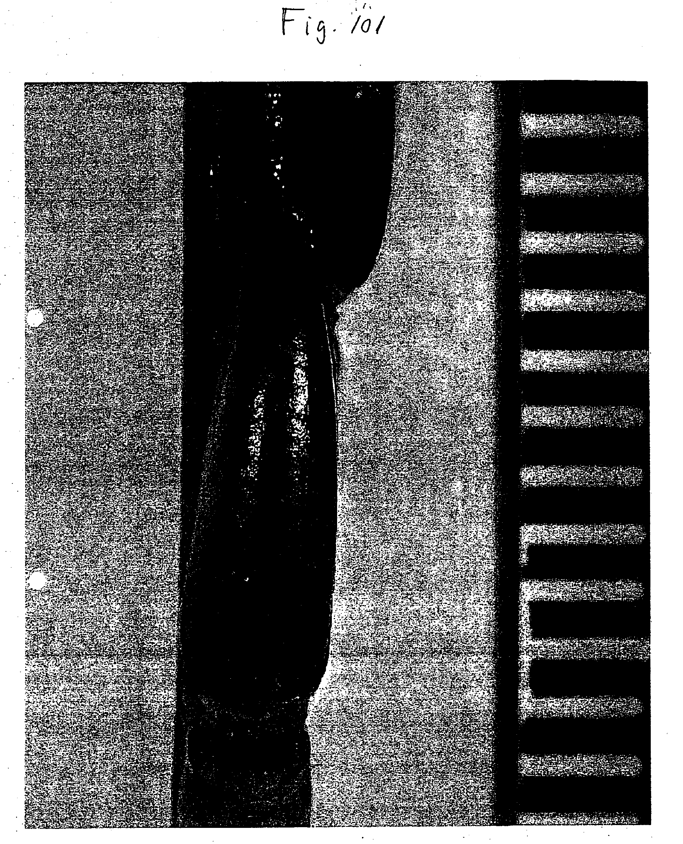

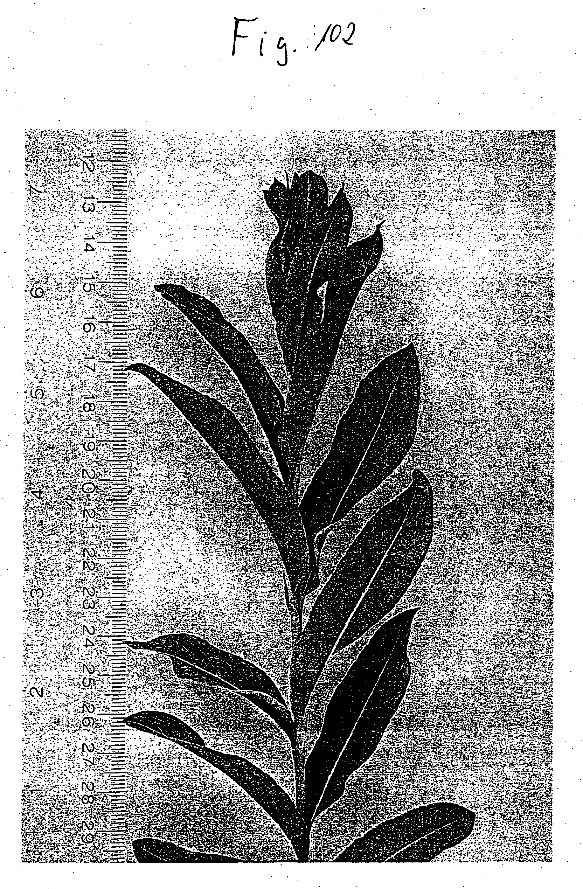

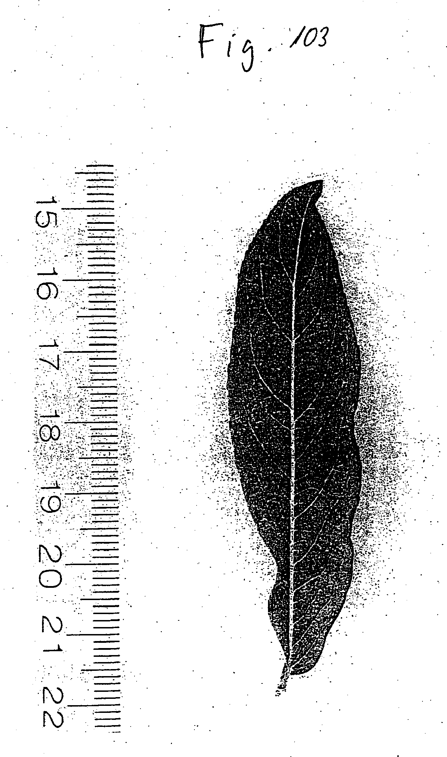

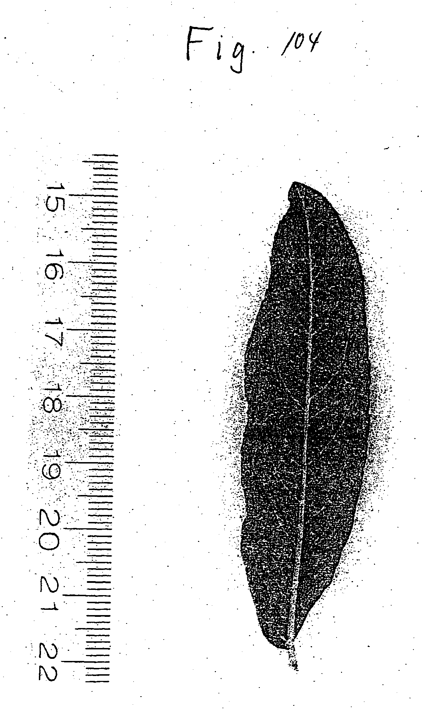

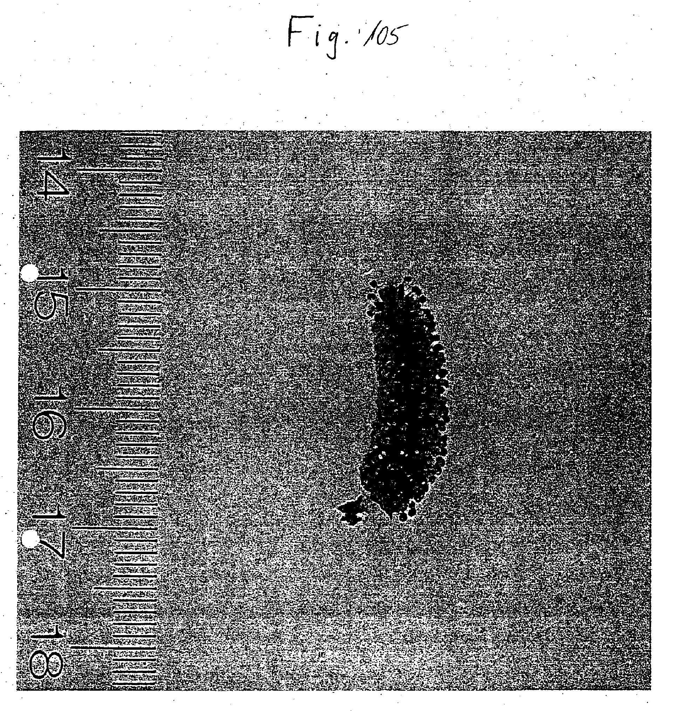

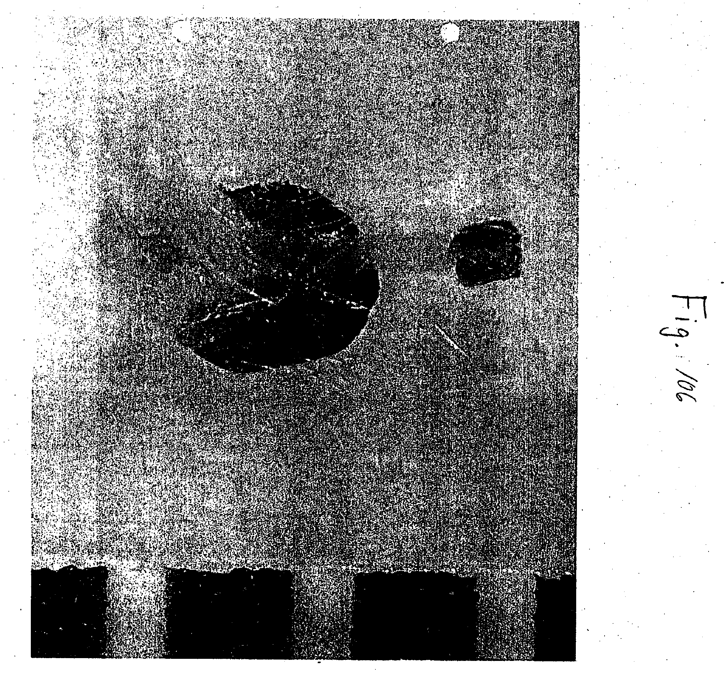

arsenic, clone D produced greater biomass and had greater transpiration rates, on average, than all other clones. However, biomass production and transpiration was reduced by approximately 60% and 75%, respectively, for clone D in the presence of arsenic without the addition of phosphate, demonstrating the extreme sensitivity of this clone to arsenic. Interestingly, symptoms of arsenic toxicity were apparent within 24 hours of the first treatment for clone D suggesting rapid uptake and translocation of arsenic (Figure 1).
The addition of phosphate to the solution had a significant effect on arsenic toxicity. The impacts of arsenic on biomass production and transpiration of the more As-sensitive clones were ameliorated by the addition of phosphate to the solution. While there were signs of arsenic toxicity even, in the presence of phosphorus for clone D, the toxic effects observed for the As 100 -P and As250 -P treatments were clearly alleviated by the addition of phosphate (Figure 1). This finding is consistent with previous reports that have shown that arsenate and phosphate are taken up via the same transport system, which has a greater affinity for phosphate (Meharg and Macnair. 1990; Mkandawire *et al.,* 2004). Therefore, with the addition of phosphate, arsenic toxicity should be reduced as a result of the competition of these compounds for the same transport pathway.
Arsenic concentrations also differed significantly among clones for all tissues (Table 2). Arsenic concentrations were generally greater in leaves than stems, and greatest in roots. Arsenic concentrations in leaves ranged from 2 to 329 µg g⁻¹ and 2 to 201 µg g⁻¹ in stems, with the greatest arsenic concentrations achieved by the most tolerant clone B. Overall aboveground arsenic content was also highest for this clone,
reflecting its relatively greater biomass production and higher arsenic concentrations compared to other clones (Figure 2). Surprisingly, for each clone, aboveground arsenic accumulation was greatest for plants that received phosphate during the treatment period (Figure 2, Table 2). These results suggest that the addition of phosphate may not only reduce arsenic toxicity, but may also play a role in enhancing arsenic uptake. The need to develop efficient and cost-effective strategies for remediation of Aspolluted soil and water is clear. Current physical and chemical methods of remediation are not only prohibitively costly, they are destructive and impractical on a large scale. Phytoremediation offers a promising alternative for the remediation of contaminated soil and water. The results presented here add to the growing body of evidence demonstrating the potential of shrub willow for phytoremediation applications.
**Clone identification:**
A = ESF clone ID# 99113-012 (*Salix purpurea*)
B = 'Tully Champion' (ESF clone ID# 99202-011, *Salix viminalis x S. miyabeana*)
C = 'Canastota' (ESF clone ID# 9970-036 *Salix sachalinensis x S. miyabeana*)
D = ESF clone ID# 00x-026-082 (*Salix eriocephala*)
**Literature cited**
Agency for Toxic Substances and Disease Registry (ATSDR). 2005. Toxicological profile for arsenic. Atlanta, GA: U.S. Department of Health and Human Services, Public Health Service.
Ebbs, S., J. Bushey, S. Poston, D. Kosma, M. Samiotakis and D. Dzombak. 2003. Transport and metabolism of free cyanide and iron cyanide complexes by willow. Plant, Cell, and the Environment. 26: 1467-1478.
E.P.A. 2003. Draft Report on the Environment. United States Environmental Protections Agency, http://www.epa.gov/indicators/roe/html/roePDF.htm.
Fitz, W. J. and W. W. Wenzel. 2002. Arsenic transformations in the soil-rhizosphereplant system: fundamentals and potential application for phytoremediation. Journal of Biotechnology 99: 259-278.
Flathman, P.E., and G.R. Lanza. 1998. Phytoremediation: current views on an emerging green technology. Journal of Soil Contamination. 7(4): 415-432.
G.A.O. 1987. Superfund: Extent of the Nation's Potential Hazardous Waste Problem Still Unknown. United States General Accounting Office, GQO/RCED 88-44, http://archive.gao.gov/d30t5/134840.pdf.
Kopp, R.F., Smart, L.B., Maynard, C.A., Isebrands, J.G., Tuskan, G.A. and L.P. Abrahamson. 2001. The development of improved willow clones for eastern North America. The Forestry Chronicle. 77 (2): 287-292.
Kuzovkina, Y. A., Knee, M. and M. F. Quigley. 2004. Cadmium and copper uptake in five willow (Salix L.) species. International journal of phytoremediation 6(3): 269-287.
Landberg, T. and M. Greger. 1996. Differences in uptake and tolerance to heavy metals in Salix viminalis. Journal of Plant Physiology 159: 69-75.
Matschullat, J. 2000. Arsenic in the geosphere- a review. The Science of the Total Environment 249: 297-312.
Meharg, A. and M. Macnair. 1990. An altered phosphate uptake system in arsenate-tolerant Holcus lanatus L. New phytologist 116: 29-35.
Mkandawire, M., Lyubum, Y. V., Kosterin, P.V. and E. G. Dudel. 2004. Toxicity of arsenic species to Lemna gibba L. and the influence of phosphate on arsenic bioavailability. Environmental Toxicology 19: 26-34.
Pulford, I. D., Riddell-Black, D. and C. Stewart. 2002. Heavy metal uptake by willow clones from sewage sludge-treated soil: The potential for phytoremediation. International Journal of Phytoremediation 4: 59-72.
Salt, D.E., M. Blaylock, N. Kumar, V. Dushenkov, B.D. Ensley, I. Chet, and I. Raskin. 1995. Phytoremediation: A novel strategy for the removal of toxic metals from the environment using plants. Biotechnology 13: 468-473.
Smart, L.B., Volk, T.A., Lin, J., Kopp, R.F., Phillips, I.S., Cameron, K.D., White, E.H. and L.P. Abrahamson. 2005. Genetic improvement of shrub willow (Salix spp.) crops for bioenergy and environmental applications in the United States. Unasylva 221 (56): 51-55.
White, E. H., Neuhauser, L. P., Abrahamson, L. P., Volk, T. A., Nowak, C. A., Peterson, J. M. Gray, E., Demeter, C. and C. Lindsey. 1999. Progress towards making willow biomass crops the fuel of the future in the northeastern United States. Proceedings of the Fourth Biomass Conference of America's Biomass: A Growth Opportunity in Green Energy and Value-Added Products, Oakland, CA, August 26-29, 1999.
The following disclosure concerns specific varieties of shrub willow that have been used or that expected to be useful in performing the inventive method. The names of the following shrub willow varieties are "Tully Champion", "Canastota", "Owasco", "Otisco", "Oneida", "Millbrook" and "Fish Creek".

### FAST-GROWING SHRUB WILLOW NAMED 'TULLY CHAMPION'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001;"Fast-Growing Shrub Willow Named 'Canastota', identified by Attorney docket number 1279-002; "Fast-Growing Shrub Willow Named 'Millbrook', identified by Attorney docket number 1279-003; "Fast-Growing Shrub Willow Named 'Oneida', identified by Attorney docket number 1279-004; "Fast-Growing Shrub Willow Named 'Otisco', identified by Attorney docket number 1279-005; and "Fast-Growing Shrub Willow Named 'Owasco', identified by Attorney docket number 1279-006. The variety of fast-growing shrub willow named 'Tully Champion' was produced in the willow breeding program at the State University of New York College of Environmental Science and Forestry, as were other varieties, including: 'Fish Creek', 'Canastota', 'Millbrook', 'Oneida', 'Otisco', and 'Owasco'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Tully Champion' resulting from the novel hybridization of *Salix viminalis* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix viminalis* x *Salix miyabeana* was the seedling progeny of the controlled pollination of the female clone *Salix viminalis* 'SV2' by the male clone *Salix miyabeana* 'SX67' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

Both parents were originally transferred from the University of Toronto (Toronto, Ontario, Canada) to the SUNY College of Environmental Science and Forestry and were vegetatively propagated from stem cuttings. The female parent (*S. viminalis* 'SV2') was transferred in 1990, while the male parent (*Salix miyabeana* 'SX67') was transferred in 1994. The growth of the parent plants was characterized in nursery plantings at the Tully Genetics Field Station owned by the SUNY College of Environmental Science and Forestry. The male clone *Salix miyabeana* 'SX67' displayed rapid stem growth and low incidence of rust disease, so was chosen to serve as a parent in a cross with *S. viminalis* 'SV2', which suffered from susceptibility to the potato leafhopper (*Empoasca fabae*). The seedlings produced by this cross (identification #99202) were first established in a greenhouse, and then were transplanted to a field at the LaFayette Road Experiment Station in Syracuse, NY owned by the SUNY College of Environmental Science and Forestry. This particular individual (identification #99202-011) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix viminalis* x *Salix miyabeana* cultivar 'Tully Champion' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Tully Champion'. These characteristics in combination distinguish 'Tully Champion' as a new and distinct cultivar:
1. Rapid growth rate, producing greater than 25% more woody biomass than two current production clones (*Salix dasyclados* 'SV1' and *Salix miyabeana* 'SX64'), more than 2.5-fold greater biomass than one of its parents (*Salix miyabeana* 'SX67'), and nearly 3-fold more biomass than another production clone (*Salix sacchalinensis,* 'SX61') when grown in the same field for the same length of time (two growing seasons after coppice) in Tully, NY.
2. Resistance to potato leafhopper, which causes severe stunting of growth, curling of the leaves, and overall decline in vigor (all characteristic of hopper burn) on the female parent, *S. viminalis* 'SV2'.
3. Low incidence of rust disease assessed in experimental trials at the LaFayette Road Experiment Station in Syracuse, NY in 2000.

### BRIEF DESCRIPTION OF THE DRAWING

The accompanying color photographs show the features of the claimed cultivar in a manner as true as is reasonably possible. The illustrations include:
Fig. 1.1 illustrates two-year-old portion of stem collected while dormant;
Fig. 1.2 illustrates one-year-old portion of stem collected while dormant;
Fig. 1.3 illustrates a vegetative bud in dormancy;
Fig. 1.4 illustrates a floral bud in dormancy;
Fig. 1.5 illustrates new shoot growth from a stem cutting rooted in soil:sand in a greenhouse;
Fig. 1.6 illustrates upper leaf surface;
Fig. 1.7 illustrates lower leaf surface;
Fig 1.8 illustrates mature catkin; and
Fig. 1.9 illustrates pistil and densely pubescent floral bract.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Tully Champion' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes every 2 hours five times each day.

Latin Name: *Salix viminalis* x *S. miyabeana*

Varietal Denomination: 'Tully Champion'

Parentage:
Female or seed parent: *S. viminalis* 'SV2'
Male or pollen parent: *S. miyabeana* 'SX67'

Propagation:
Type: Stem cutting
Time to rooting: Approximately 10 days in water at 21°C

Precocity: Precocious -Catkins mature several days before leaves break bud.

Plant description: The color of one-year-old stem cuttings observed when dormant are grey orange (RHS 168B), while two to three-year-old stems are yellow-green (RHS 152C), lustrous, and glaucous in some places. Vegetative buds are red (RHS 168B), ovoid, and acute in two to three-year-old stems, and elongated in one-year-old growth with pubescence behind the buds. Lenticels are reddish brown to tan, large, wart-like, and numerous. The leaves are simple and alternate with pinnate venation. Petioles are typically 4mm in length. Stipules are typically 3-4 mm in length, narrow, curved, and serrulate. Immature leaves have a few small hairs. Mature leaves are lanceolate, acuminate apex, acute base, typically 7.5-9.0 cm in length, 1.0-1.5 cm in width, sometimes undulate, and serrate margin, adaxial (upper) surface green (RHS 144B), pale green abaxial (lower) surface (RHS 145A), and light pale green stem (RHS 145B) at 7 ½ weeks of growth.

Flowering description: Dormant floral buds are elongated, ovoid, acute, beak-like apex, typically 9 mm in length, slightly raised to form a very small acute angle with the stem, and red (N34A). Peduncle of catkin is short and bears 4 leafy bracts. Catkins are erect, typically 3.6 mm in length, narrowly cylindrical, and densely flowered. Flowers have a pubescent medium sized sessile ovary with a medium length style and 2 erect, slightly separated stigmas. Floral bract is densely pubescent with a dark acute apex and a pink base.

Field growth characteristics: Determined through surveys of plants growing in the field at SUNY-ESF's Tully Genetics Field Station in Tully, NY and at LaFayette Road Experiment Station in Syracuse, NY.

Disease resistance: Displays a low incidence of rust disease.

Temperature tolerance: Stems typically do not suffer frost damage at temperatures as low as 10°F and may suffer only minor tip dieback at lower temperatures.

Seed production: 'Tully Champion' produces only female flowers, so viable seeds will only be produced after pollination by a compatible male variety. This has not yet been observed in field trials.

Biomass yield: Mean dry stem biomass yield produced through two growing seasons after coppice in each of eight four-plant plots ('Tully Champion', 14.02 oven dry tons ha⁻¹ yr⁻¹) measured in a yield trial growing at the Tully Genetics Field Station in Tully, NY in February 2005 was 2.5-fold greater than the mean stem biomass yield of one of its parents ('SX67', 5.52 oven dry tons ha⁻¹ yr⁻¹); was more than 25% greater than current production cultivars ('SV1', 11.04 oven dry tons ha⁻¹ yr⁻¹ and 'SX64', 10.35 oven dry tons ha⁻¹ yr⁻¹); and was nearly 3-fold greater than the mean stem biomass yield of another production cultivar ('SX61'; 4.83 oven dry tons ha⁻¹ yr⁻¹) growing in the same trial (Fig 2.1).

### CLAIM

What is claimed is:
A new and distinct variety of cultivar of *Salix viminalis* x *S. miyabeana* named 'Tully Champion', substantially as shown and described, which grows rapidly to produce significantly greater dry stem biomass than one of its parents ('SX67') and of other production cultivars ('SV1', 'SX64' and 'SX61').

### ABSTRACT OF THE DISCLOSURE

A distinct female cultivar of *Salix viminalis* x *S. miyabeana* named 'Tully Champion', characterized by rapid stem growth producing greater than 25% more woody biomass than two current production clones (*Salix dasyclados* 'SV1' and *Salix miyabeana* 'SX64'), more than 2.5-fold greater biomass than one of its parents (*Salix miyabeana* 'SX67'), and nearly 3-fold more biomass than another production clone *(Salix sacchalinensis,* 'SX61') when grown in the same field for the same length of time (two growing seasons after coppice) in Tully, NY. 'Tully Champion' can be planted from dormant stem cuttings, produces multiple stems after coppice, and the stem biomass can be harvested when the plant is dormant. In the spring following harvest, the plant will re-sprout very vigorously, producing new stems that can be harvested repeatedly after two to four years of growth. 'Tully Champion' displays a low incidence of rust disease and is not damaged by potato leafhoppers.

### FAST-GROWING SHRUB WILLOW NAMED 'CANASTOTA'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001; "Fast-Growing Shrub Willow Named 'Millbrook', identified by Attorney docket number 1279-003; "Fast-Growing Shrub Willow Named 'Oneida', identified by Attorney docket number 1279-004; "Fast-Growing Shrub Willow Named 'Otisco', identified by Attorney docket number 1279-005; "Fast-Growing Shrub Willow Named 'Owasco', identified by Attorney docket number 1279-006; and "Fast-Growing Shrub Willow Named 'Tully Champion', identified by Attorney docket number 1279-007. The variety of fast-growing shrub willow named 'Canastota' was produced in the willow breeding program at the State University of New York College of Environmental Science and Forestry, as were other varieties, including: 'Fish Creek', 'Millbrook', 'Oneida', 'Otisco', 'Owasco', and 'Tully Champion'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Canastota' resulting from the novel hybridization of *Salix sachalinensis* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix sachalinensis* x *S. miyabeana* was the seedling progeny of the controlled pollination of the female clone *Salix sachalinensis* 'SX61' by the male clone *Salix miyabeana* 'SX64' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

Both parents (*Salix sachalinensis* 'SX61' and *Salix miyabeana* 'SX64' were originally transferred from the University of Toronto (Toronto, Ontario, Canada) to the SUNY College of Environmental Science and Forestry in 1994 and were vegetatively propagated from stem cuttings. The growth of the parent plants was characterized in experimental studies at the Tully Genetics Field Station owned by the SUNY College of Environmental Science and Forestry. Both parents displayed rapid stem growth and low incidence of rust disease, so were chosen to serve as parents in a cross. The seedlings produced by this cross (cross identification # 9970) were first established in a greenhouse, and then were transplanted to a field at the LaFayette Road Experiment Station in Syracuse, NY owned by the SUNY College of Environmental Science and Forestry. This particular individual (individual #9970-036) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix sachalinensis* x *S. miyabeana* cultivar 'Canastota' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Canastota'. These characteristics in combination distinguish 'Canastota' as a new and distinct cultivar:
1. Rapid growth rate, producing greater than 2.7-fold more woody biomass than its female parent (*Salix sachalinensis* 'SX61'), 28% greater woody biomass yield than its male parent (*Salix miyabeana* 'SX64'), and 20% greater woody biomass yield than a standard production cultivar, *Salix dasyclados* 'SV1' when grown in the same field for the same length of time (two growing seasons after coppice) in Tully, NY.
2. Low incidence of rust disease or willow sawfly damage as assessed in nursery plantings in Tully, NY in October, 2004.

### BRIEF DESCRIPTION OF THE DRAWING

The accompanying color photographs show the features of the claimed cultivar in a manner as true as is reasonably possible. The illustrations include:
Fig. 1.1 illustrates two-year-old portion of stem collected while dormant;
Fig. 1.2 illustrates one-year-old portion of stem collected while dormant;
Fig. 1.3 illustrates a vegetative bud in dormancy;
Fig. 1.4 illustrates a floral bud in dormancy;
Fig. 1.5 illustrates new shoot growth from a stem cutting rooted in soil:sand in a greenhouse;
Fig. 1.6 illustrates upper leaf surface;
Fig. 1.7 illustrates lower leaf surface;
Fig 1.8 illustrates mature catkin;
Fig. 1.9 illustrates stamen and densely pubescent floral bract; and
Fig. 1.10 illustrates lenticels.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Canastota' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes at 2 hour intervals five times each day.

Latin Name: *Salix sachalinensis* x *S. miyabeana*

Varietal Denomination: 'Canastota'

Parentage:
Female or seed parent: *S. sachalinensis* 'SX61'
Male or pollen parent: *S. miyabeana* 'SX64'

Propagation:
Type: Stem cuttings
Time to rooting: Approximately 10 days in water at 21°C

Precocity: Subprecocious - Catkins mature as leaves begin to break bud.

Plant description: The color of one-year-old stems observed when dormant is typically grey orange (RHS 171A), while two-year-old stems are yellow-green (RHS 152B) with bark beginning to crack and the cuticle broken and textured. Dormant vegetative buds are dark red (RHS 180A), 4.5 mm in length, narrow, and acute. Lenticels are peach colored and randomly scattered, 0.5-1 mm in diameter. The leaves are simple and alternate with pinnate venation. Petioles are typically 4mmin length. Stipules are lanceolate, serrate, curved, and typically 3-4 mm in length. Pubescent immature leaves. Mature leaves are oblong, acute apex, acute to obtuse base, typically 8.5-12.0 cm in length, 1.7-2.1 cm in width, serrate margin, adaxial (upper) surface green (RHS 144A), abaxial (lower) surface pale green (RHS 143C), and stem light pale green (RHS 144D) at 5 weeks of growth.

Flowering description: Dormant floral buds are ovate, diameter rounded, beak-like apex, slightly raised to form a very small acute angle with stem , typically 10 mm in length, bright dark red (RHS 60A). Peduncle of catkin is short and bears four leafy bracts. Catkins are curved 90-180°, typically 2.8 mm in length, broadly cylindrical, and densely flowered. Flowers have one stamen, and one nectary, a densely pubescent floral bract with a green base and pink-purple obtuse apex, long united filament two times the length of the floral bract, and small anthers.

Field growth characteristics: Determined through surveys of plants growing in the field at SUNY-ESF's Tully Genetics Field Station in Tully, NY.

Disease and pest resistance: Surveys completed in October 2004 in Tully, NY indicate no to low levels of detectable rust incidence and no to low incidence of sawfly or beetle damage.

Temperature tolerance: Stems typically do not suffer frost damage at temperatures as low as 10°F and may suffer only minor tip dieback at lower temperatures.

Seed production: None -does not produce female flowers

Biomass yield: Mean total dry stem biomass expressed as oven dry tons (odt) per hectare (ha) and year (yr) produced through two growing seasons after coppice in four 4-plant plots ('Canastota', 13.3 odt ha⁻¹ yr⁻¹) measured in a yield trial growing at the Tully Genetics Field Station in Tully, NY in February 2005 was greater than the mean biomass yield of either parent ('SX61', 4.8 odt ha⁻¹ yr⁻¹; 'SX64', 10.4 odt ha⁻¹ yr⁻¹) and was greater than a current production cultivar ('SV1', 11.0 odt ha⁻¹ yr⁻¹) growing in the same trial (Fig 2.1).

### CLAIM

What is claimed is:
A new and distinct cultivar of *Salix sachalinensis* x *S. miyabeana* named 'Canastota', substantially as shown and described, which grows rapidly to produce significantly greater dry stem biomass than either of its parents or the standard production cultivar *Salix dasyclados* 'SV1'.

### ABSTRACT OF THE DISCLOSURE

A distinct male cultivar of *Salix sachalinensis* x *S. miyabeana* named 'Canastota', characterized by rapid stem growth producing greater than 2.7-fold more woody biomass than its female parent (*Salix sachalinensis* 'SX61'), 28% greater woody biomass yield than its male parent (*Salix miyabeana* 'SX64'), and 20% greater woody biomass yield than a standard production cultivar, *Salix dasyclados* 'SV1' when grown in the same field for the same length of time (two growing seasons after coppice) in Tully, NY. 'Canastota' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. 'Canastota' displays a low incidence of rust disease or damage by willow sawfly.

### FAST-GROWING SHRUB WILLOW NAMED 'OWASCO'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001; "Fast-Growing Shrub Willow Named 'Canastota', identified by Attorney docket number 1279-002; "Fast-Growing Shrub Willow Named 'Millbrook', identified by Attorney docket number 1279-003; "Fast-Growing Shrub Willow Named 'Oneida', identified by Attorney docket number 1279-004; "Fast-Growing Shrub Willow Named 'Otisco', identified by Attorney docket number 1279-005; and "Fast-Growing Shrub Willow Named 'Tully Champion', identified by Attorney docket number 1279-007. The variety of fast-growing shrub willow named 'Owasco' was produced in the willow breeding program at the State University of New York College of Environmental Science and Forestry, as were other varieties, including: 'Fish Creek', 'Canastota', 'Millbrook', 'Oneida', 'Otisco', and 'Tully Champion'.

### STATEMENT AS TO RIGHTS TO INVENTIONS MADE UNDER FEDERALLY-SPONSORED RESEARCH AND DEVELOPMENT (IF ANY)

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Owasco' resulting from the novel hybridization of *Salix viminalis* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix viminalis* x *Salix miyabeana* was the seedling progeny of the controlled pollination of the female clone *Salix viminalis* 'SV7' by the male clone *Salix miyabeana* 'SX64' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

Both parents were originally transferred from the University of Toronto (Toronto, Ontario, Canada) to the SUNY College of Environmental Science and Forestry and were vegetatively propagated from stem cuttings. The female parent (*S. viminalis* 'SV7') was transferred in 1990, while the male parent (*Salix miyabeana* 'SX64') was transferred in 1994. The growth of the parent plants was characterized in nursery plantings at the Tully Genetics Field Station owned by the SUNY College of Environmental Science and Forestry. The male clone *Salix miyabeana* 'SX64' displayed rapid stem growth and low incidence of rust disease, so was chosen to serve as a parent in a cross with *S. viminalis* 'SV7', which suffered from susceptibility to the potato leafhopper (*Empoasca fabae*). The seedlings produced by this cross (identification #99207) were first established in a greenhouse, and then were transplanted to a field at the LaFayette Road Experiment Station in Syracuse, NY owned by the SUNY College of Environmental Science and Forestry. This particular individual (identification #99207-018) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix viminalis* x *S. miyabeana* cultivar 'Owasco' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Owasco'. These characteristics in combination distinguish 'Owasco' as a new and distinct cultivar:
1. Rapid growth rate, producing greater than 49% more woody biomass than one of its parents (*Salix miyabeana* 'SX64'), 39% more biomass than one current production cultivar (*Salix dasyclados* 'SV1'), and more than 2.7-fold more biomass that two other production cultivars (*Salix miyabeana* 'SX67' and *Salix sachalinensis* 'SX61') when grown in the same field for the same length of time (two growing seasons after coppice) in Tully, NY.
2. Resistance to potato leafhopper, which causes severe stunting of growth, curling of the leaves, and overall decline in vigor (all characteristic of hopper burn) on the female parent, *S. viminalis* 'SV7'.
3. Low incidence of rust disease assessed in experimental trials at the LaFayette Road Experiment Station in Syracuse, NY in 2000.

### BRIEF DESCRIPTION OF THE DRAWING

The accompanying color photographs show the features of the claimed cultivar in a manner as true as is reasonably possible. The illustrations include:
Fig. 1.1 illustrates two-year-old portion of stem collected while dormant;
Fig. 1.2 illustrates one-year-old portion of stem collected while dormant;
Fig. 1.3 illustrates a vegetative bud in dormancy;
Fig. 1.4 illustrates a floral bud in dormancy;
Fig. 1.5 illustrates new shoot growth from a stem cutting rooted in soil:sand in a greenhouse;
Fig. 1.6 illustrates upper leaf surface;
Fig. 1.7 illustrates lower leaf surface;
Fig. 1.8 illustrates mature catkin; and
Fig. 1.9 illustrates pistil and densely pubescent floral bract.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Owasco' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes every 2 hours five times each day.

Latin Name: *Salix viminalis* x *S. miyabeana*

Varietal Denomination: 'Owasco'

Parentage:
Female or seed parent: *S. viminalis* 'SV7'
Male or pollen parent: *S. miyabeana* 'SX64'

Propagation:
Type: Stem cutting
Time to rooting: Approximately 10 days in water at 21°C

Precocity: Subprecocious - Catkins mature as leaves begin to break bud.

Plant description: The color of one-year-old stem cuttings observed when dormant are grey orange (RHS 171 A), while two to three-year-old stems are yellow-green (RHS 152C) smooth, lustrous, and glaucous. Vegetative buds are red-orange (RHS N172B), ovoid, acute, and typically 3.5-4.0 mm in length. Lenticels are large, wart-like, numerous, brown to red, and lighter in the center. The leaves are simple and alternate with pinnate venation. Petioles are typically 3mm in length. Stipules are typically 2 mm in length, ovate, and slightly curved, and serrulate. Immature leaves have a few small hairs. Mature leaves are lanceolate, acuminate apex, acute base, typically 7.5-9.0 cm in length, 1.4-1.8 cm in width, serrulate margin, adaxial (upper) surface green (RHS 144A), abaxial (lower) surface pale green (RHS 144B), and stem light pale green (RHS 144C) at 7 ½ weeks of growth.

Flowering description: Dormant floral buds are elongated, acute, ovoid, beak-like apex, typically 8.5 mm in length, appressed, and red (RHS 180B). Peduncle of catkin is typically 4 mm in length and bears 5 leafy bracts. Catkins are narrowly cylindrical, erect, typically 2.4 cm in length, and densely flowered. Flowers have a short, narrow, densely hairy ovary on a pedicel with a very long style (length of ovary) and 2 long and curled stigmas. Floral bract is densely pubescent, pink, and obtuse.

Field growth characteristics: Determined through surveys of plants growing in the field at SUNY-ESF's Tully Genetics Field Station in Tully, NY and at LaFayette Road Experiment Station in Syracuse, NY.

Disease resistance: Displays a low incidence of rust disease.

Temperature tolerance: Stems typically do not suffer frost damage at temperatures as low as 10°F and may suffer only minor tip dieback at lower temperatures.

Seed production: 'Owasco' produces only female flowers, so viable seeds will only be produced after pollination by a compatible male variety. This has not yet been observed in field trials.

Biomass yield: Mean dry stem biomass yield produced through two growing seasons after coppice in each of eight four-plant plots ('Owasco', 15.45 oven dry tons ha⁻¹ yr⁻¹) measured in a yield trial growing at the Tully Genetics Field Station in Tully, NY in February 2005 was 49% greater than the mean stem biomass yield of one of its parents ('SX64', 10.35 oven dry tons ha⁻¹ yr⁻¹) and was 39% greater than a current production cultivar ('SV1', 11.04 oven dry tons ha⁻¹ yr⁻¹) growing in the same trial (Fig 2.1). 'Owasco' produced greater than 2.7-fold more stem biomass than two other current production cultivars ('SX67', 5.52 oven dry tons ha⁻¹ yr⁻¹; 'SX61', 4.83 oven dry tons ha⁻¹ yr⁻¹) growing in the same trial (Fig. 2.1).

### CLAIM

What is claimed is:
A new and distinct variety of cultivar of *Salix viminalis* x *Salix miyabeana* named 'Owasco', substantially as shown and described, which grows rapidly to produce significantly greater dry stem biomass than one of its parents ('SX64') and of other production cultivars ('SV1', 'SX67' and 'SX61').

### ABSTRACT OF THE DISCLOSURE

A distinct female cultivar of *Salix viminalis* x *Salix miyabeana* named 'Owasco', characterized by rapid stem growth producing greater than 49% more woody biomass than one of its parents ('SX64') and 39% more biomass than a current production cultivar ('SV1'). 'Otisco' produced greater than 2.7-fold more stem biomass than two other current production cultivars, 'SX67' and 'SX61'. 'Owasco' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. The stem biomass can be chipped and burned as a source of renewable energy, generating heat and/or electricity. 'Owasco' displays a low incidence of rust disease and is not damaged by potato leafhoppers.

### FAST-GROWING SHRUB WILLOW NAMED 'OTISCO'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001; "Fast-Growing Shrub Willow Named 'Canastota', identified by Attorney docket number 1279-002; "Fast-Growing Shrub Willow Named 'Millbrook', identified by Attorney docket number 1279-003; "Fast-Growing Shrub Willow Named 'Oneida', identified by Attorney docket number 1279-004; "Fast-Growing Shrub Willow Named 'Owasco', identified by Attorney docket number 1279-006; and "Fast-Growing Shrub Willow Named 'Tully Champion', identified by Attorney docket number 1279-007. The variety of fast-growing shrub willow named 'Otisco' was produced in the willow breeding program at the State University of New York College of Environmental Science and Forestry, as were other varieties, including: 'Fish Creek', 'Canastota', 'Millbrook', 'Oneida', 'Owasco', and 'Tully Champion'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Otisco' resulting from the novel hybridization of *Salix viminalis* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix viminalis* x *Salix miyabeana* was the seedling progeny of the controlled pollination of the female clone *S. viminalis* 'SV2' by the male clone *S. miyabeana* 'SX64' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

Both parents were originally transferred from the University of Toronto (Toronto, Ontario, Canada) to the SUNY College of Environmental Science and Forestry and were vegetatively propagated from stem cuttings. The female parent (*S. viminalis* 'SV2') was transferred in 1990, while the male parent (*Salix miyabeana* 'SX64') was transferred in 1994. The growth of the parent plants was characterized in nursery plantings at the Tully Genetics Field Station owned by the SUNY College of Environmental Science and Forestry. The male clone *Salix miyabeana* 'SX64' displayed rapid stem growth and low incidence of rust disease, so was chosen to serve as a parent in a cross with *S. viminalis* 'SV2', which suffered from susceptibility to the potato leafhopper (*Empoasca fabae*). The seedlings produced by this cross (identification #99201) were first established in a greenhouse, and then were transplanted to a field at the LaFayette Road Experiment Station in Syracuse, NY owned by the SUNY College of Environmental Science and Forestry. This particular individual (identification #99201-007) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix viminalis* x *S. miyabeana* cultivar 'Otisco' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Otisco'. These characteristics in combination distinguish 'Otisco' as a new and distinct cultivar:
1. Rapid growth rate, producing greater than 42% more woody biomass than one of its parents (*Salix miyabeana* 'SX64'), 33% more biomass than one current production cultivar (*Salix dasyclados* 'SV1'), and more than 2.5-fold more biomass that two other production cultivars (*Salix miyabeana* 'SX67' and *Salix sachalinensis* 'SX61') when grown in the same field for the same length of time (two growing seasons after coppice) in Tully, NY.
2. Resistance to potato leafhopper, which causes severe stunting of growth, curling of the leaves, and overall decline in vigor (all characteristic of hopper burn) on the female parent, *S. viminalis* 'SV2'.
3. Low incidence of rust disease assessed in experimental trials at the LaFayette Road Experiment Station in Syracuse, NY in 2000.

### BRIEF DESCRIPTION OF THE DRAWING

The accompanying color photographs show the features of the claimed cultivar in a manner as true as is reasonably possible. The illustrations include:
Fig. 1.1 illustrates two-year-old portion of stem collected while dormant;
Fig. 1.2 illustrates one-year-old portion of stem collected while dormant;
Fig. 1.3 illustrates a vegetative bud in dormancy;
Fig. 1.4 illustrates a floral bud in dormancy;
Fig. 1.5 illustrates new shoot growth from a stem cutting rooted in soil:sand in a greenhouse;
Fig. 1.6 illustrates upper leaf surface;
Fig. 1.7 illustrates lower leaf surface;
Fig. 1.8 illustrates mature catkin; and
Fig. 1.9 illustrates pistil and densely pubescent floral bract.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Otisco' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes every 2 hours five times each day.

Latin Name: *Salix viminalis* x *S. miyabeana*

Varietal Denomination: 'Otisco'

Parentage:
Female or seed parent: *S. viminalis* 'SV2'
Male or pollen parent: *S. miyabeana* 'SX64'

Propagation:
Type: Stem cutting
Time to rooting: Approximately 10 days in water at 21°C

Precocity: Subprecocious - Catkins mature as leaves begin to break bud.

Plant description: The color of one-year-old stem cuttings observed when dormant are grey orange (RHS 167B), while two to three-year-old stems are yellow-green (RHS 153B) to cracking pale green (RHS 195B) bark. Vegetative buds are red-orange (RHS 179A), obtuse, linear, pubescent, and typically 3.5-4 mm in length. Lenticels are red, large, wart-like, and numerous. The leaves are simple and alternate with pinnate venation. Stipules are lanceolate, serrulate, slightly curved, and typically 4 mm in length. Immature leaves are pubescent. Mature leaves are lanceolate, acuminate apex, acute base, typically 8.5-9.6 cm in length, 1.1-1.8 cm in width, serrulate margin, adaxial (upper) surface green (RHS 144A), abaxial (lower) surface pale green (RHS 143D), and stem light pale green (RHS 145B) at 6 weeks of growth.

Flowering description: Dormant floral buds are elongated, ovoid, acute, typically 9 mm in length, appressed, and red-orange (RHS 171 A). Peduncle of catkin is typically 3.5 mm in length and bears 4 leafy bracts. Catkins are erect, typically 2.8 mm in length, narrowly cylindrical, and densely flowered. Flowers have a long, narrow, densely hairy, and sessile ovary, with a long style and 2 stigmas one larger than the other. Floral bract is densely pubescent and has a pink acute apex.

Field growth characteristics: Determined through surveys of plants growing in the field at SUNY-ESF's Tully Genetics Field Station in Tully, NY and at LaFayette Road Experiment Station in Syracuse, NY.

Disease resistance: Displays a low incidence of rust disease.

Temperature tolerance: Stems typically do not suffer frost damage at temperatures as low as 10°F and may suffer only minor tip dieback at lower temperatures.

Seed production: 'Otisco' produces only female flowers, so viable seeds will only be produced after pollination by a compatible male variety. This has not yet been observed in field trials.

Biomass yield: Mean dry stem biomass yield produced through two growing seasons after coppice in each of eight four-plant plots ('Otisco', 14.78 oven dry tons ha⁻¹ yr⁻¹) measured in a yield trial growing at the Tully Genetics Field Station in Tully, NY in February 2005 was 42% greater than the mean stem biomass yield of one of its parents ('SX64', 10.35 oven dry tons ha⁻¹ yr⁻¹) and was 33% greater than a current production cultivar ('SV1', 11.04 oven dry tons ha⁻¹ yr⁻¹) growing in the same trial (Fig 2.1). 'Otisco' produced greater than 2.5-fold more stem biomass than two other current production cultivars ('SX67', 5.52 oven dry tons ha⁻¹ yr⁻¹; 'SX61', 4.83 oven dry tons ha⁻¹ yr⁻¹) growing in the same trial (Fig. 2.1).

### CLAIM

What is claimed is:
A new and distinct variety of cultivar of *Salix viminalis* x *S. miyabeana* named 'Otisco', substantially as shown and described, which grows rapidly to produce significantly greater dry stem biomass than one of its parents ('SX64') and of other production cultivars ('SV1', 'SX67' and 'SX61').

### ABSTRACT OF THE DISCLOSURE

A distinct female cultivar of *Salix viminalis* x *S. miyabeana* named 'Otisco', characterized by rapid stem growth producing greater than 42% more woody biomass than one of its parents ('SX64') and 33% more biomass than a current production cultivar ('SV1'). 'Otisco' produced greater than 2.5-fold more stem biomass than two other current production cultivars, 'SX67' and 'SX61'. 'Otisco' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. The stem biomass can be chipped and burned as a source of renewable energy, generating heat and/or electricity. 'Otisco' displays a low incidence of rust disease and is not damaged by potato leafhoppers.

### FAST-GROWING SHRUB WILLOW NAMED 'ONEIDA'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001; "Fast-Growing Shrub Willow Named 'Canastota', identified by Attorney docket number 1279-002; "Fast-Growing Shrub Willow Named 'Millbrook', identified by Attorney docket number 1279-003; "Fast-Growing Shrub Willow Named 'Otisco', identified by Attorney docket number 1279-005; "Fast-Growing Shrub Willow Named 'Owasco', identified by Attorney docket number 1279-006; and "Fast-Growing Shrub Willow Named 'Tully Champion', identified by Attorney docket number 1279-007. The variety of fast-growing shrub willow named 'Oneida' was produced in the willow breeding program at the State University of New York College of Environmental Science and Forestry, as were other varieties, including: 'Fish Creek', 'Canastota', 'Millbrook', 'Otisco', 'Owasco', and 'Tully Champion'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Oneida' resulting from the novel hybridization of *Salix purpurea* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix purpurea* x *S. miyabeana* was the seedling progeny of the controlled pollination of the female clone *Salix purpurea* '94006' by the male clone *Salix miyabeana* 'SX67' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

The female parent (*Salix purpurea* '94006' was originally identified in 1994 by personnel employed by the SUNY College of Environmental Science and Forestry growing on the shore of a creek in Oneida County, New York and was vegetatively propagated from stem cuttings. The male parent *(Salix miyabeana* 'SX67') was originally transferred from the University of Toronto (Toronto, Ontario, Canada) to the SUNY College of Environmental Science and Forestry in 1994 and was vegetatively propagated from stem cuttings. The growth of the parent plants was characterized in experimental studies at the Tully Genetics Field Station owned by the SUNY College of Environmental Science and Forestry. Both parents displayed rapid stem growth and low incidence of rust disease, so were chosen to serve as parents in a cross (identification # 9980). The seedlings produced by this cross were first established in a greenhouse, and then were transplanted to a field at the LaFayette Road Experiment Station in Syracuse, NY owned by the SUNY College of Environmental Science and Forestry. This particular individual (identification #9980-005) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix purpurea* x *S. miyabeana* cultivar 'Oneida' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Oneida'. These characteristics in combination distinguish 'Oneida' as a new and distinct cultivar:
1. Rapid growth rate, producing 2.7-fold more woody biomass than one of its parents *(Salix miyabeana* 'SX67'), 45% more biomass than cultivar *Salix miyabeana* 'SX64', and 36% more biomass than a standard production cultivar, *Salix dasyclados* 'SV1' when grown in the same field for the same length of time (two growing seasons after coppice) in Tully, NY.
2. Low incidence of rust disease or mammal browse damage assessed in experimental trials at the LaFayette Road Experiment Station in Syracuse, NY in 2000.

### BRIEF DESCRIPTION OF THE DRAWING

The accompanying color photographs show the features of the claimed cultivar in a manner as true as is reasonably possible. The illustrations include:
Fig. 1.1 illustrates two-year-old portion of stem collected while dormant;
Fig. 1.2 illustrates one-year-old portion of stem collected while dormant;
Fig. 1.3 illustrates a vegetative bud in dormancy;
Fig. 1.4 illustrates a floral bud in dormancy;
Fig. 1.5 illustrates new shoot growth from a stem cutting rooted in soil:sand in a greenhouse;
Fig. 1.6 illustrates upper leaf surface;
Fig. 1.7 illustrates lower leaf surface;
Fig 1.8 illustrates mature catkin;
Fig. 1.9 illustrates stamen and densely pubescent floral bract; and
Fig. 1.10 illustrates double vegetative buds.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Oneida' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes every 2 hours five times each day.

Latin Name: *Salix purpurea* x *S. miyabeana*

Varietal Denomination: 'Oneida'

Parentage:
Female or seed parent: *S. purpurea* '94006'
Male or pollen parent: *S. miyabeana* 'SX67'

Propagation:
Type: Stem cutting
Time to rooting: Approximately 10 days in water at 21°C

Precocity: Subprecocious - Catkins mature as leaves begin to break bud.

Plant description: The color of one-year-old stems observed when dormant are grey orange (RHS 171A), while two to three-year-old stems are yellow-green to pale green (RHS 152C, RHS 147C), bark cracking and cuticle slightly broken vertically. Vegetative buds are dark red (RHS 60A), typically 3.5mm in length, and lingulate, occasionally occurring together as double buds. Lenticels are wart-like, randomly scattered, and red to yellow. The leaves are simple and alternate with pinnate venation. Petioles are typically 5 mm in length. Stipules are typically 5 mm in length, lanceolate, and serrulate. Pubescent immature leaves. Mature leaves are oblanceolate, acute apex, acute to obtuse base, typically 8.1-11.5 cm in length, 1.0-1.3 cm in width, serrulate margin, adaxial (upper) surface green (RHS 144A), abaxial (lower) surface light pale green (RHS 139C), and stem light pale green (RHS 145C), at five weeks of growth.

Flowering description: Dormant floral buds are elongated, ovoid, acute, abaxial surface rounded, adaxial surface flat, typically 11mm in length, raised forming an acute angle with the stem, turned, and dark brown. Peduncle of catkin is short and bears 3 to 4 leafy bracts. Catkins are erect, approximately 3.3 mm in length, and densely flowered. Flowers have one stamen, one nectary, a densely pubescent floral bract with a pink-purple acute apex, a long united filament two times the length of the floral bract, and dark anthers with bright yellow pollen.

Field growth characteristics: Determined through surveys of plants growing in the field at SUNY-ESF's Tully Genetics Field Station in Tully, NY and at LaFayette Road Experiment Station in Syracuse, NY.

Disease resistance: Displays a low incidence of rust disease; minor incidence of stem canker disease is occasionally evident.

Temperature tolerance: Stems typically do not suffer frost damage at temperatures as low as 10°F and may suffer only minor tip dieback at lower temperatures.

Seed production: None -does not produce female flowers.

Biomass yield: Mean dry stem biomass yield produced through two growing seasons after coppice in eight 4-plant plots ('Oneida', 15.04 oven dry tons ha⁻¹ yr⁻¹) measured in a yield trial growing at the Tully Genetics Field Station in Tully, NY in February 2005 was 2.7-times greater than the mean stem biomass yield of one of its parents ('SX67', 5.52 oven dry tons ha⁻¹ yr⁻¹) and was more than 36% greater than two current production cultivars ('SV1', 11.04 oven dry tons ha⁻¹ yr⁻¹ and 'SX64', 10.35 oven dry tons ha⁻¹ yr⁻¹) growing in the same trial (Fig 2.1).

### CLAIM

What is claimed is:
A new and distinct variety of cultivar of *Salix purpurea* x *S. miyabeana* named 'Oneida', substantially as shown and described, which grows rapidly to produce significantly greater dry stem biomass than one of its parents ('SX67') and of other production cultivars ('SV1' and 'SX64').

### ABSTRACT OF THE DISCLOSURE

A distinct male cultivar of *Salix purpurea* x *S. miyabeana* named 'Oneida', characterized by rapid stem growth producing 2.7-times greater woody biomass than one of its parents ('SX67') and greater than 36% more biomass than current production cultivars ('SV1' and 'SX64'). 'Oneida' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. The stem biomass can be chipped and burned as a source of renewable energy, generating heat and/or electricity. 'Oneida' displays a low incidence of rust disease or damage by beetles or sawflies.

### FAST-GROWING SHRUB WILLOW NAMED 'MILLBROOK'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Fish Creek', identified by Attorney docket number 1279-001; "Fast-Growing Shrub Willow Named 'Canastota', identified by Attorney docket number 1279-002; "Fast-Growing Shrub Willow Named 'Oneida', identified by Attorney docket number 1279-004; "Fast-Growing Shrub Willow Named 'Otisco', identified by Attorney docket number 1279-005; "Fast-Growing Shrub Willow Named 'Owasco', identified by Attorney docket number 1279-006; and "Fast-Growing Shrub Willow Named 'Tully Champion', identified by Attorney docket number 1279-007. The variety of fast-growing shrub willow named 'Millbrook' was produced in the willow breeding program at the State University of New York College of Environmental Science and Forestry, as were other varieties, including: 'Fish Creek', 'Canastota', 'Oneida', 'Otisco', 'Owasco', and 'Tully Champion'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar known by the varietal name 'Millbrook' resulting from the novel hybridization of *Salix purpurea* with *Salix miyabeana.* The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing. Shrub willow is being developed as an agricultural crop plant that will be grown and harvested as a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix purpurea* x *Salix miyabeana* was the seedling progeny of the controlled pollination of the female clone *Salix purpurea* '95026' by the male clone *Salix miyabeana* 'SX64' performed in February 1999 on the campus of the SUNY College of Environmental Science and Forestry in Syracuse, NY. The plant has been propagated repeatedly by stem cuttings and has been found to retain its distinctive characteristics through successive propagations and field trials.

The female parent, *Salix purpurea* '95026' was originally identified in 1995 growing on the shore of a creek in Dutchess County, New York and was vegetatively propagated from stem cuttings. The male parent, *Salix miyabeana* 'SX64' was originally transferred from the University of Toronto (Toronto, Ontario, Canada) to the SUNY College of Environmental Science and Forestry in 1994 and was vegetatively propagated from stem cuttings. The growth of the parent plants was characterized in nursery plantings at the Tully Genetics Field Station owned by the SUNY College of Environmental Science and Forestry. Both parents displayed rapid stem growth and low incidence of disease, so were chosen to serve as parents in a cross. The seedlings produced by this cross (identification #99217) were first established in a greenhouse, and then were transplanted to a field at the LaFayette Road Experiment Station in Syracuse, NY owned by the SUNY College of Environmental Science and Forestry. This particular individual (identification #99217-015) was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix purpurea* x *Salix miyabeana* cultivar 'Millbrook' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Millbrook'. These characteristics in combination distinguish 'Millbrook' as a new and distinct cultivar:
1. Rapid growth rate, producing 9% more woody biomass than one of its parents (*Salix miyabeana* 'SX64'), 2% more biomass than one current production cultivar (*Salix dasyclados* 'SV1'), and more than 2-fold more biomass that two other production cultivars (*Salix miyabeana* 'SX67' and *Salix sachalinensis* 'SX61') when grown in the same field for the same length of time (two growing seasons after coppice) in Tully, NY.
2. Low incidence of rust disease assessed in experimental trials at the LaFayette Road Experiment Station in Syracuse, NY in 2000.

### BRIEF DESCRIPTION OF THE DRAWING

The accompanying color photographs show the features of the claimed cultivar in a manner as true as is reasonably possible. The illustrations include:

Fig. 1.1 illustrates two-year-old portion of stem collected while dormant;

Fig. 1.2 illustrates one-year-old portion of stem collected while dormant;

Fig. 1.3 illustrates a vegetative bud in dormancy;

Fig. 1.4 illustrates a floral bud in dormancy;

Fig. 1.5 illustrates new shoot growth from a stem cutting rooted in soil:sand in a greenhouse;

Fig. 1.6 illustrates upper leaf surface;

Fig. 1.7 illustrates lower leaf surface;

Fig. 1.8 illustrates mature catkin; and

Fig. 1.9 illustrates pistil and densely pubescent floral bract.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed description of the 'Millbrook' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes every 2 hours five times each day.

Latin Name: *Salix purpurea* x *S. miyabeana*

Varietal Denomination: 'Millbrook'

Parentage:
Female or seed parent: *S. purpurea* '95026'
Male or pollen parent: *S. miyabeana* 'SX64'

Propagation:
Type: Stem cuttings
Time to rooting: Approximately 10 days in water at 21°C

Precocity: Subprecocious - Catkins mature as leaves begin to break bud.

Plant description: The color of one-year-old stem cuttings observed when dormant are grey orange (RHS 164C, RHS171A), while two- to three-year-old stems are yellow-green to pale green (RHS 153A, RHS 148D) and lustrous. Vegetative buds are red-orange (RHS 180A), deltate, and typically 4 mm in length. Lenticels are large, brown, and sparse. The leaves are simple and alternate with pinnate venation. Petioles are typically 3 mm in length. Stipules are typically 1 mm in length and ovate. Immature leaves have very few hairs. Mature leaves are slightly oblanceolate, acute apex, acute-obtuse base, typically 8.1-8.8 cm in length, 1.5-1.9 cm in width, serrate margin, adaxial (upper) surface green (144A), abaxial (lower) surface light pale green (143D), and stem light green (RHS 144C) at 6 weeks of growth.

Flowering description: Dormant floral buds are elongated, ovoid, acute, typically 9.5 mm in length, slightly raised, and red (RHS 179A). Peduncle of catkin is typically 3.5 mm in length and bears 4-5 leafy bracts. Catkins are curved 90°, typically 2.6-3.0 mm in length, and narrowly cylindrical. Flowers have a short, round, sessile ovary with short soft hairs, a very short style, and 2 broad stigmas. Floral bract is densely hairy with a pink obtuse apex.

Field growth characteristics: Determined through surveys of plants growing in the field at SUNY-ESF's Tully Genetics Field Station in Tully, NY and at LaFayette Road Experiment Station in Syracuse, NY.

Disease resistance: Displays a low incidence of rust disease.

Temperature tolerance: Stems typically do not suffer frost damage at temperatures as low as 10°F and may suffer only minor tip dieback at lower temperatures.

Seed production: 'Millbrook' produces only female flowers, so viable seeds will only be produced after pollination by a compatible male variety. This has not yet been observed in field trials.

Biomass yield: Mean dry stem biomass yield produced through two growing seasons after coppice in each of eight four-plant plots ('Millbrook', 11.30 oven dry tons ha⁻¹ yr⁻¹) measured in a yield trial growing at the Tully Genetics Field Station in Tully, NY in February 2005 was 9% greater than the mean stem biomass yield of one of its parents ('SX64', 10.35 oven dry tons ha⁻¹ yr⁻¹) and was 2% greater than a current production cultivar ('SV1', 11.04 oven dry tons ha⁻¹ yr⁻¹) growing in the same trial (Fig 2.1). 'Millbrook' produced greater than 2-fold more stem biomass than two other current production cultivars ('SX67', 5.52 oven dry tons ha⁻¹ yr⁻¹; 'SX61', 4.83 oven dry tons ha⁻¹ yr⁻¹) growing in the same trial (Fig. 2.1).

### CLAIM

What is claimed is:
A new and distinct variety of cultivar of *Salix purpurea* x *Salix miyabeana* named 'Millbrook', substantially as shown and described, which grows rapidly to produce significantly greater dry stem biomass than one of its parents ('SX64') and of other production cultivars ('SV1', 'SX67' and 'SX61').

### ABSTRACT OF THE DISCLOSURE

A distinct female cultivar of *Salix purpurea* x *Salix miyabeana* named 'Millbrook', characterized by rapid stem growth producing 9% more woody biomass than one of its parents ('SX64') and 2% more biomass than a current production cultivar ('SV1'). 'Millbrook' produced greater than 2-fold more stem biomass than two other current production cultivars, 'SX67' and 'SX61'. 'Millbrook' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. The stem biomass can be chipped and burned as a source of renewable energy, generating heat and/or electricity. 'Millbrook' displays a low incidence of rust disease.

### FAST-GROWING SHRUB WILLOW NAMED 'FISH CREEK'

### CROSS-REFERENCE TO RELATED APPLICATIONS

This application is related to the following plant patent applications, all of which are subject to assignment to the Research Foundation of the State University of New York, and each of which is being filed on even date herewith: "Fast-Growing Shrub Willow Named 'Canastota', identified by Attorney docket number 1279-002; "Fast-Growing Shrub Willow Named 'Millbrook', identified by Attorney docket number 1279-003; "Fast-Growing Shrub Willow Named 'Oneida', identified by Attorney docket number 1279-004; "Fast-Growing Shrub Willow Named 'Otisco', identified by Attorney docket number 1279-005; "Fast-Growing Shrub Willow Named 'Owasco', identified by Attorney docket number 1279-006; and "Fast-Growing Shrub Willow Named 'Tully Champion', identified by Attorney docket number 1279-007. The variety of fast-growing shrub willow named 'Fish Creek' was produced in the willow breeding program at the State University of New York College of Environmental Science and Forestry, as were other varieties, including: 'Canastota', 'Millbrook', 'Oneida', 'Otisco', 'Owasco', and 'Tully Champion'.

### STATEMENT AS TO RIGHTS TO INVENTIONS MADE UNDER FEDERALLY-SPONSORED RESEARCH AND DEVELOPMENT

The invention described herein was reduced to practice during the funding period of Contract 4000003235 (SUNY Research Foundation Award 011275) awarded by Oak Ridge National Laboratory, managed by UT-Batelle for the United States Department of Energy under contract DE-AC05-00OR22725, and of agreement number 6267 (SUNY Research Foundation Award 011536) awarded by the New York State Energy Research and Development Authority.

### BACKGROUND OF THE INVENTION

### 1. FIELD OF THE INVENTION

The invention is a new and distinct cultivar of *Salix purpurea* known by the varietal name 'Fish Creek'. The new variety was produced through controlled willow breeding conducted by the inventors at the State University of New York College of Environmental Science and Forestry in Syracuse, NY. The objective of the breeding program is to produce new willow cultivars that generate high biomass yields on a variety of sites, are resistant to diseases and pests, and possess agronomic traits suitable for mechanical planting, harvesting, and post-harvest processing to provide a sustainable, renewable source of energy. Once a field planting of shrub willows is established, the woody stems can be harvested every three years, and new shoots will re-sprout the following season. Repeated harvesting every two to four years can be sustained for at least 15 years.

### 2. DESCRIPTION OF RELEVANT PRIOR ART INCLUDING INFORMATION DISCLOSED UNDER 37 CFR 1.97 - 1.99.

This new variety of *Salix purpurea* was the seedling progeny of the controlled pollination of a female clone (ID# 94006) of *Salix purpurea* by a male clone of *Salix purpurea* (ID# 94001) performed in February 1998 on the campus of the SUNY College of Environmental Science and Forestry in Syracuse, NY. The plant has been propagated repeatedly by stem cuttings and has been found to retain its distinctive characteristics through successive propagations and field trials.

Both parents (94006 and 94001) were originally identified in 1994 growing on the shores of creeks in Oneida County, New York and were vegetatively propagated from stem cuttings. The growth of the parent plants was characterized in nursery plantings at the Tully Genetics Field Station owned by the SUNY College of Environmental Science and Forestry. Both parents displayed rapid stem growth and low incidence of rust disease, so were chosen to serve as parents in a cross. The seedlings produced by this cross (identification #9882) were first established in a greenhouse, and then were transplanted to a field at the LaFayette Road Experiment Station in Syracuse, NY owned by the SUNY College of Environmental Science and Forestry. This particular individual (identification #9882-34) shown in the Figures was selected from the family due to its exceptional stem height growth.

The new cultivar has been grown in Syracuse, NY and Tully, NY, which have a normal yearly average daily temperature of 47°F, normal daily maximum temperature in July of 82°F, normal daily minimum temperature in January of 14°F, and average precipitation of 40 inches. The new cultivar grows from a rooted cutting to a fully mature plant ready for harvest in approximately three years.

### SUMMARY OF THE INVENTION

The *Salix purpurea* cultivar 'Fish Creek' has not been observed under all possible environmental conditions. The phenotype may vary somewhat with variations in environments such as temperature, light intensity and length of illumination, without, however, any variation in genotype. The new and distinct cultivar presents the following traits that have been repeatedly observed and are determined to be the unique characteristics of 'Fish Creek'. These characteristics in combination distinguish 'Fish Creek' as a new and distinct cultivar:
1. Rapid growth rate, producing greater than 30% more woody biomass than its parents when grown in the same field for the same length of time (three growing seasons after coppice) in Tully, NY.
2. Low incidence of rust disease or damage by beetle or sawfly as scored by visual inspection.

### BRIEF DESCRIPTION OF THE DRAWING

The accompanying color photographs show the features of the claimed cultivar in a manner as true as is reasonably possible. The illustrations include:

Fig. 1.1 illustrates one-year-old portion of stem collected while dormant;

Fig. 1.2 illustrates one-year-old portion of stem collected while dormant;

Fig. 1.3 illustrates a vegetative bud in dormancy;

Fig. 1.4 illustrates a floral bud in dormancy;

Fig. 1.5 illustrates new shoot growth from a stem cutting rooted in sand:potting mix in a greenhouse;

Fig. 1.6 illustrates upper leaf surface;

Fig. 1.7 illustrates lower leaf surface;

Fig. 1.8 illustrates catkin with anthers; and

Fig. 1.9 illustrates stamen and densely pubescent bud scale.

### DETAILED DESCRIPTION OF THE NEW PLANT

The following detailed description sets forth characteristics of the new plant. The following observations and measurements describe plants grown by asexual reproduction in Syracuse, NY or Tully, NY under conditions as described hereinabove. Color references are made using The Royal Horticultural Society Colour Chart (hereinafter the R.H.S. Colour Chart) of The Royal Horticultural Society of London, England, except where general terms of ordinary dictionary significance are used.

### BOTANICAL DESCRIPTION OF THE PLANT

The following detailed botanical description of the 'Fish Creek' variety is based on observations from 10 inch cuttings grown in a greenhouse in Syracuse, NY. Cuttings were grown in 7 inch tubes in a (1:1) ProMix®/sand (v/v) substrate under natural light from December 2004 to March 2005. Plants were irrigated with automatic misting for 6 minutes at 2 hours intervals five times each day.

Latin Name: *Salix purpurea*

Varietal Denomination: 'Fish Creek'

Parentage:
Female or seed parent: *S. purpurea* '94006'
Male or pollen parent: *S. purpurea* '94001'

Propagation:
Type: Stem cuttings
Time to rooting: Approximately 10 days in water at 21°C

Precocity: Precocious - Catkins mature several days before leaves break bud.

Plant description: The color of one-year-old stems observed when dormant is typically brown (RHS 177A) on the newest portions, while older portions are smooth, glaucous, and green (RHS 146C). Dormant vegetative buds produced during the previous growing season are yellow to orange (RHS 162, RHS 171 A), 6 mm in length, ovoid, and acute. Lenticels are small, sparse, reddish, and 1 mm in diameter. The leaves are simple and opposite to subopposite (occasionally alternate) with pinnate venation. Petioles are typically 2 mm in length. There are no stipules. Immature leaves are glabrous. Mature leaves are oblong, obtuse apex, obtuse base, typically 6.1 - 6.9 cm in length, 1.6 - 2.1 cm in width, with slightly serrate to subentire margins, curved, adaxial (upper) surface dark green (RHS 143A) with glaucous flakes, abaxial (lower) surface light pale green (RHS 144B), and stem light pale green (RHS 145B) at 7 ½ weeks of growth.

Flowering description: Dormant floral buds are elongated, acute, 11 mm in length, appressed, diameter rounded, and brown (RHS 165A) to golden-brown (RHS 165B). Peduncle of catkin is short and bears four leaf-like bracts, approximately 4 mm in length. Catkins are erect, 2.2 - 2.4 mm in length, narrowly cylindrical, and densely flowered. Flowers have one stamen and one nectary, a densely pubescent floral bract, thick filament, orange immature anthers, and yellow-brown mature anthers.

Field growth characteristics: Determined through surveys of plants growing in the field at SUNY-ESF's Tully Genetics Field Station in Tully, NY.

Disease and insect resistance: Leaves display low incidence of beetle damage and stems display low incidence of sawfly damage - scored as a 1 (on a scale of 1 to 3, with 1 being the least amount of damage and 3 the greatest) in a field surveys conducted in August and September 2001. Leaves are largely resistant to rust as scored by visual inspection at the end of each growing season.

Temperature tolerance: Stems typically do not suffer frost damage at temperatures as low as 10°F and may suffer only minor tip dieback at lower temperatures.

Seed production: None - does not produce female flowers.

Stem growth and biomass yield: Mean total area of stems produced through one growing season after coppice in each of three 20 plant plots ('Fish Creek', 142 cm²) measured in a yield trial growing at the Tully Genetics Field Station in Tully, NY in April 2003 was greater than the mean stem area of either parent ('94001', 90 cm²; '94006', 114 cm²) and was greater than a current production cultivar ('SV1', 104 cm²) growing in the same trial (Fig. 2.1).

Total dry stem biomass yield ('Fish Creek' 53,500 kg per hectare) harvested after three growing seasons after coppice in February and March 2005 in the same trial described above was more than 30% greater than either parent ('94001' 39,900 kg per hectare; '94006' 39,100 kg per hectare) and 20% greater than the biomass produced by a current production cultivar ('SV1' 44,400 kg per hectare) (Fig. 2.2).

### CLAIM

What is claimed is:
A new and distinct variety of cultivar of *Salix purpurea* named 'Fish Creek', substantially as shown and described, which grows rapidly to produce significantly greater dry stem biomass than either of its parents.

### ABSTRACT OF THE DISCLOSURE

A distinct male cultivar of *Salix purpurea* named 'Fish Creek', characterized by rapid stem growth producing greater than 30% more woody biomass than either of its parents ('94001' and '94006') and 20% more biomass than a current production cultivar ('SV1'). 'Fish Creek' can be planted from dormant stem cuttings, produces multiple stems after coppice, and the stem biomass can be harvested when the plant is dormant. In the spring following harvest, the plant will re-sprout very vigorously, producing new stems that can be harvested after two to four years of growth. This harvest cycle can be repeated several times. The stem biomass can be chipped and burned as a source of renewable energy, generating heat and/or electricity. 'Fish Creek' displays a low incidence of rust disease or damage by beetles or sawflies.

### Brief description of the figures

Figure 1 is a photograph concerning the role of P in reducing As.
Figure 2 is a graph concerning the total above ground As content of severals clons.
Figures 3 to 9 are bare graphs showing the biomass yield of severals cultivares.
Figure 10 and 11 are bare graphs showing the stem area of several cultivares.

The further attached 15 pages of figures 12 to 41 concern or disclose the above features, further aspects, details and examples of the prior art and of the system and method according to the invention.

The further attached 9 pages of figures 42 to 50 are the photographs accompanying the disclosure of the shrub willow named "Tully Champion".

The further attached 10 pages of figures 51 to 60 are the photographs accompanying the disclosure of the shrub willow named "Canastota".

The further attached 9 pages of figures 61 to 69 are the photographs accompanying the disclosure of the shrub willow named "Owasco".

The further attached 9 pages of figures 70 to 78 are the photographs accompanying the disclosure of the shrub willow named "Otisco".

The further attached 10 pages of figures 79 to 88 are the photographs accompanying the disclosure of the shrub willow named "Oneida".

The further attached 9 pages of figures 89 to 97 are the photographs accompanying the disclosure of the shrub willow named "Millbrook".

The further attached 9 pages of figures 98 to 106 are the photographs accompanying the disclosure of the shrub willow named "Fish Creek".

## Claims

1. Use of shrub willow for taking up and accumulating arsenic from contaminated growth medium.

2. Use according to claim 1, wherein the growth medium is soil and/or shore.

3. Use according to claim 1 or 2, wherein the contaminated growth medium comprises phosphorus and/or phosphorus is added to the growth medium.

4. Use according to any of claims 1 to 3, wherein the shrub willow is selected from the following species/cultivars: Salix purpurea, Salix viminalis x S. miyabeana, Salix sachalinensis x S. miyabeana, Salix eriocephala and crosses thereof.

5. Use according to claim 4, wherein the shrub willow is selected from the following clones:
ESF clone ID#99113-012, ESF clone ID#99202-011, ESF clone ID#9970-036, ESF clone ID#00x#026-082 and crosses thereof.

6. Use according to any of claims 1 to 5, wherein a plurality of shrub willows of the same and/or different species/cultures is used on the growth medium.

7. Use according to any of claims 1 to 6, wherein the stems and leaves with the arsenic accumulated therein are harvested and removed from the growth medium.

8. Use according to claim 7, wherein the stems and leaves are harvested every 2 to 4 years.

9. Use according to any of claims 1 to 8, wherein at least part of the contaminated shrub willow is disposed off in a secure landfill.

10. Use according to any of claims 1 to 9, wherein at least part of the contaminated shrub willow is burned as biomass and the contaminant is collected in the ash remaining after incineration of the plant tissues and/or is scrubbed from the effluent gases generated during the burning of the biomass.
